(19) Europäisches Patentamt / European Patent Office / Office européen des brevets

(11) **EP 4 640 678 A1**

(12) **EUROPEAN PATENT APPLICATION**
published in accordance with Art. 153(4) EPC


(43) Date of publication:
**29.10.2025 Bulletin 2025/44**

(21) Application number: **23906002.3**

(22) Date of filing: **20.12.2023**

(51) International Patent Classification (IPC):
***C07D 401/10*** *(2006.01)* ***A61K 31/4439*** *(2006.01)*
***A61P 9/10*** *(2006.01)* ***A61P 9/00*** *(2006.01)*
***A61P 7/02*** *(2006.01)*

(52) Cooperative Patent Classification (CPC):
Y02P 20/55

(86) International application number:
**PCT/CN2023/140251**

(87) International publication number:
**WO 2024/131841 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.12.2022 CN 202211647390**



(71) Applicant: **Chengdu Shibeikang Biomedical Technology Co., Ltd.**
**Chengdu, Sichuan 611731 (CN)**

(72) Inventors:
- **ZENG, Yanqun**
  **Chengdu, Sichuan 611731 (CN)**
- **ZHU, Xucheng**
  **Chengdu, Sichuan 611731 (CN)**
- **HUANG, Long**
  **Chengdu, Sichuan 611731 (CN)**
- **ZHOU, Guanglin**
  **Chengdu, Sichuan 611731 (CN)**
- **FU, Haixia**
  **Chengdu, Sichuan 611731 (CN)**

(74) Representative: **Herrero & Asociados, S.L.**
**Cedaceros, 1**
**28014 Madrid (ES)**


(54) **NOVEL OXO-PYRIDINE COMPOUND, PREPARATION METHOD THEREFOR, AND USE THEREOF**


(57) Disclosed in the present invention are a compound as represented by formula (I), a stereoisomer or pharmaceutically acceptable salt thereof, and a key intermediate thereof. The present invention also provides a use of the compound, and the stereoisomer or pharmaceutically acceptable salt thereof in the preparation of drugs for treating and/or preventing FXIa receptor-related diseases, especially a use in the preparation of drugs for treating and/or preventing cerebrovascular arterial diseases and/or peripheral arterial diseases.

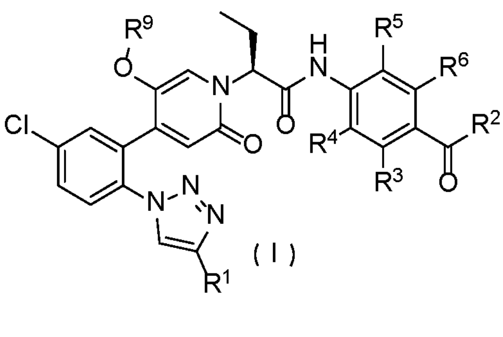

( I )


EP 4 640 678 A1

## Description

CROSS-REFERENCE TO RELATED APPLICATION

The present application claims priority to Invention Patent No. CN 202211647390.2, filed on December 21, 2022, and entitled "NOVEL OXO-PYRIDINE COMPOUND, PREPARATION METHOD THEREFOR, AND USE THEREOF", which is explicitly incorporated herein by reference.

TECHNICAL FIELD

The present disclosure relates to the field of pharmaceutical chemistry, and in particular to an oxo-pyridine compound or a salt or isomer thereof, a preparation method therefor and the use thereof in the preparation of drugs for treating and/or preventing FXIa receptor-associated diseases, especially for use in the preparation of drugs for treating and/or preventing cerebrovascular arterial diseases and/or peripheral arterial diseases or the like.

BACKGROUND ART

Thromboembolism is a disease caused by abnormal blood clots formed in blood vessels of living humans and animals. Thrombosis is caused by three factors: vessel injury, changes in blood composition, and blood stasis. It is a group of complications resulting from various diseases and reasons. Due to variations in underlying diseases and differences in thromboembolic locations, thrombotic disorders may be clinically manifested as myocardial infarction, stroke, deep vein thrombosis (DVT), pulmonary embolism, atrial fibrillation, cerebral infarction or the like. Notably, myocardial infarction, cerebral infarction and pulmonary infarction triggered mainly by embolism and infarction are leading causes of death, claiming nearly 12 million lives worldwide each year, almost a quarter of the world's total deaths.

Coagulation factor XI (FXI) is a plasma serine proteinase zymogen essential for maintaining endogenous pathways. Upon activation, it converts into activated coagulation factor XIa (FXIa), which plays a key role during the amplification of the coagulation cascade. In the coagulation cascade, thrombin can feedback activate FXI, and the activated FXI subsequently promotes the production of a significant amount of thrombin, thereby amplifying the coagulation cascade. Therefore, drugs targeting FXI can block the intrinsic pathway and suppress the amplification of the coagulation cascade, thus having an antithrombotic effect. In recent years, the clinical data regarding the correlation between human coagulation factor XI (FXI) deficiency or elevation in FXI level and the occurrence of thrombotic diseases, coupled with antithrombotic experimental studies in animals with FXI deficiency, knockout or inhibition show that, compared with a direct FXa inhibitor, the inhibition of FXI may offer a reduced bleeding risk. Therefore, FXI is a new target for antithrombotic prevention and treatment.

Reported FXI inhibitors mainly include monoclonal antibodies, antisense oligonucleotides, small chemical molecules, polypeptides or proteins, and peptidomimetics. Currently, milvexian, developed jointly by BMS and Johnson & Johnson, has completed Phase II clinical trials, showing a significantly lower risk of bleeding. The Phase I clinical trial of BMS's small-molecule FXIa inhibitor BMS-962122 for intravenous injection has been completed, but the research and development have been halted. The small-molecule oral FXIa inhibitor ONO-7684, developed by Ono Pharmaceutical Co., Ltd. (Japan), has entered Phase I clinical trials. Monoclonal antibodies and antisense oligonucleotides need to be administered by injection, and have limitations such as high cost, delayed onset of therapeutic effect, and possible challenges in control.

The efficacy, pharmacokinetics and toxicity of drugs in organisms are influenced by a variety of factors and also exhibit corresponding complexity. Minor changes in chemical structure at any position, such as deuteration, may cause changes in the efficacy, pharmacokinetics and toxicity in vivo. Compared with corresponding non-deuterated drugs, the variation of the pharmacokinetics and other properties of deuterated drugs exhibits great contingency and unpredictability. Deuteration at some sites has the effect of extending the half-life, e.g., marketed donafenib tosylate and deuterated tetrabenazine, while deuteration at some sites can not extend but shorten the half-life (Scott L. Harbeson, Roger D. Tung. Deuterium in Drug Discovery and Development, P405-406). In addition, the hydrogen at some sites on drug molecules cannot be deuterated easily due to steric hindrance, making the deuteration sites of a drug unpredictable.

Asundexian, an FXI inhibitor from Bayer, has entered Phase III clinical trial, with an average terminal half-life of up to 17.8 h, which is a long half-life, and a risk of bleeding, which is superior to apixaban to some degree but still relatively high. Therefore, the research and development of a novel small-molecule FXIa inhibitor drug with stronger activity, better absorption, relatively rapid metabolism, lower risk of bleeding and better safety can compensate for the shortcomings of current clinical anticoagulant and antithrombotic drugs that are prone to bleeding complications and meet the unmet clinical needs.

## SUMMARY OF THE INVENTION

The compound of the present disclosure is a novel oxo-pyridine compound, and the compounds in most examples demonstrate a good anticoagulant effect and have a strong inhibitory effect on FXIa.

In one aspect, the present disclosure provides a compound represented by formula (I), or a stereoisomer or pharmaceutically acceptable salt thereof:

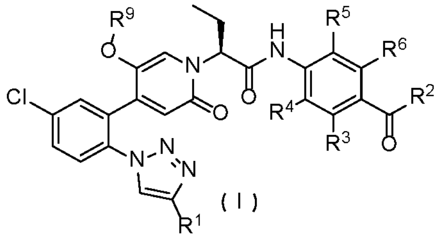

(I)

wherein

$R^1$ is selected from halogen or trifluoromethyl;
$R^2$ is independently selected from $NH_2$, $NHR^7$, OH or $OR^8$, wherein $R^7$ and $R^8$ are selected from alkyl or cycloalkyl, and hydrogen of $R^7$ and $R^8$ may be substituted with one or more deuterium;
$R^3$ and $R^6$ are independently selected from hydrogen, halogen, alkoxy, haloalkyl or $CONH_2$;
$R^4$ and $R^5$ are independently selected from hydrogen, halogen, alkoxy or haloalkyl; and
$R^9$ is independently selected from $CH_3$ or $CD_3$; and
wherein the following compounds are excluded:

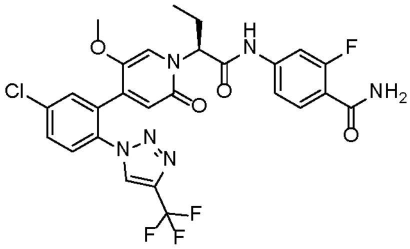
,
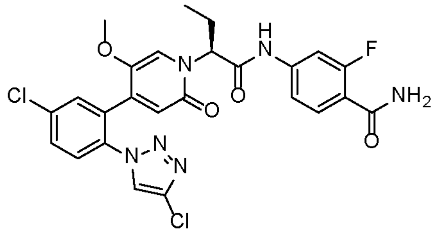
,

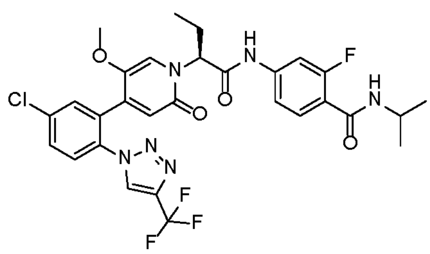
.

Further, in the above compound, or the stereoisomer or pharmaceutically acceptable salt thereof, the $\mathbf{R^1}$ is selected from chloro or trifluoromethyl;

or/and $R^2$ is independently selected from $NH_2$, $NHR^7$, OH or $OR^8$, wherein $R^7$ and $R^8$ are selected from methyl, ethyl, propyl, cyclopropyl, cyclopropylmethyl or tert-butyl, and hydrogen of $R^7$ and $R^8$ may be substituted with one or more deuterium;
or/and $R^3$ and $R^6$ are independently selected from hydrogen, fluoro, chloro, methoxy, trifluoromethyl or $CONH_2$;
or/and $R^4$ and $R^5$ are independently selected from hydrogen, fluoro, chloro, methoxy or trifluoromethyl;
or/and $R^9$ is independently selected from $CH_3$ or $CD_3$.

Further, in the above compound, or the stereoisomer or pharmaceutically acceptable salt thereof, the $R^1$ is selected from trifluoromethyl;

or/and $R^2$ is independently selected from $NH_2$, $NHR^7$, OH or $OR^8$, wherein $R^7$ and $R^8$ are selected from methyl, ethyl, propyl, cyclopropyl, cyclopropylmethyl or tert-butyl, and hydrogen of $R^7$ and $R^8$ may be substituted with one or more deuterium;
or/and $R^3$ and $R^6$ are independently selected from hydrogen, fluoro, chloro, methoxy, trifluoromethyl or $CONH_2$;
or/and $R^4$ and $R^5$ are independently selected from hydrogen, fluoro or chloro;
or/and $R^9$ is independently selected from $CH_3$ or $CD_3$.

Further, in the above compound, or the stereoisomer or pharmaceutically acceptable salt thereof, the $R^1$ is selected from trifluoromethyl;

or/and $R^2$ is independently selected from $NH_2$, $NHCH_3$, $NHCD_3$, OH, $OCH_3$, $OCD_3$ or $OC(CH_3)_3$;
or/and $R^3$ and $R^6$ are independently selected from fluoro, chloro, methoxy or trifluoromethyl;
or/and $R^4$ and $R^5$ are independently selected from hydrogen, fluoro or chloro;
or/and $R^9$ is independently selected from $CH_3$ or $CD_3$.

Further, the above compound, or the stereoisomer or pharmaceutically acceptable salt thereof includes the following structures:

| No. | Structure | Name |
|---|---|---|
| Compound 1 | 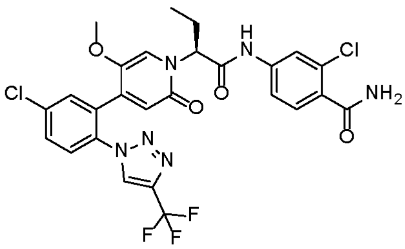 | (S)-2-chloro-4-(2-(4-(5-chloro-2-(4-(trifluoromethyl)-1H-1,2,3-triazol-1-yl)phenyl)-5-methoxy-2-oxopyridin-1(2H)-yl)butanamido)benzamide |
| Compound 2 | 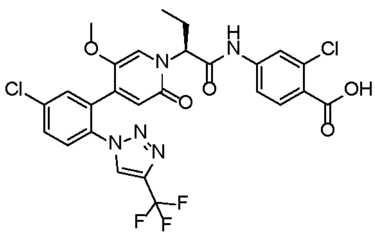 | (S)-2-chloro-4-(2-(4-(5-chloro-2-(4-(trifluoromethyl)-1H-1,2,3-triazol-1-yl)phenyl)-5-methoxy-2-oxopyridin-1(2H)-yl)butanamido)benzoic acid |
| Compound 3 | 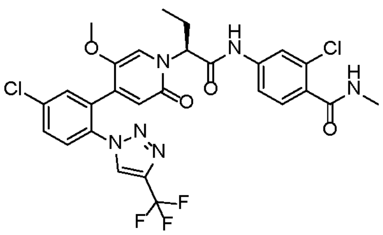 | (S)-2-chloro-4-(2-(4-(5-chloro-2-(4-(trifluoromethyl)-1H-1,2,3-triazol-1-yl)phenyl)-5-methoxy-2-oxopyridin-1(2H)-yl)butanamido)-N-methylbenzamide |
| Compound 4 | 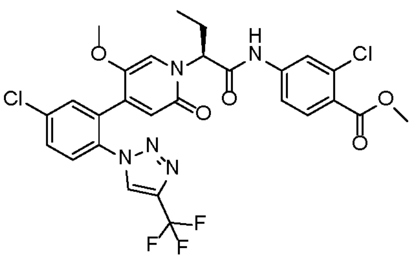 | Methyl (S)-2-chloro-4-(2-(4-(5-chloro-2-(4-(trifluoromethyl)-1H-1,2,3-triazol-1-yl)phenyl)-5-methoxy-2-oxopyridin-1(2H)-yl)butanamido)benzoate |

(continued)

| No. | Structure | Name |
|---|---|---|
| Compound 5 | 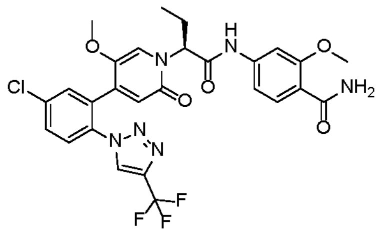 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2-methoxybenzamide |
| Compound 6 | 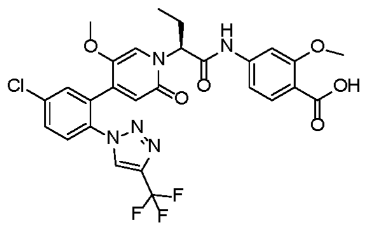 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2-methoxybenzoic acid |
| Compound 7 | 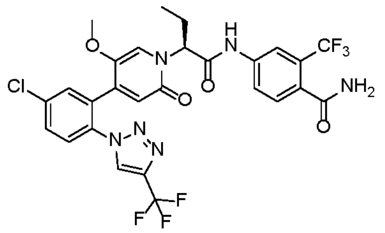 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2-(trifluoromethyl)benzamide |
| Compound 8 | 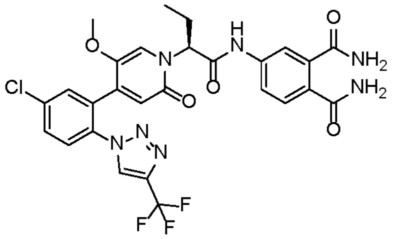 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)phthalamide |
| Compound 9 | 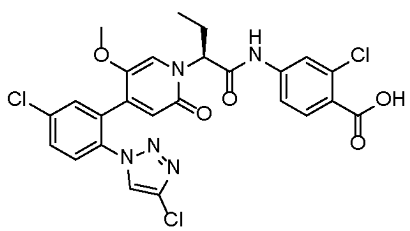 | (S)-2-chloro-4-(2-(4-(5-chloro-2-(4-chloro-1H-1,2,3-triazol-1-yl)phenyl)-5-methoxy-2-oxopyridin-1(2H)-yl)butanamido)benzoic acid |
| Compound 10 | 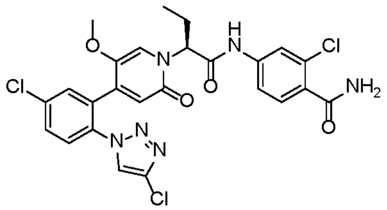 | (S)-2-chloro-4-(2-(4-(5-chloro-2-(4-chloro-1H-1,2,3-triazol-1-yl)phenyl)-5-methoxy-2-oxopyridin-1(2H)-yl)butanamido)benzamide |
| Compound 11 | 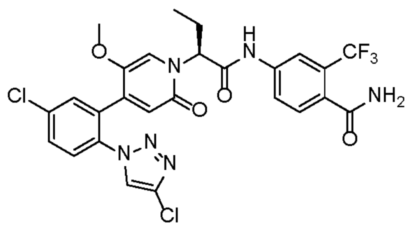 | (S)-4-(2-(4-(5-chloro-2-(4-chloro-1H-1,2,3-triazol-1-yl)phenyl)-5-methoxy-2-oxopyridin-1(2H)-yl)butanamido)-2-(trifluoromethyl)benzamide |

(continued)

| No. | Structure | Name |
|---|---|---|
| Compound 12 | 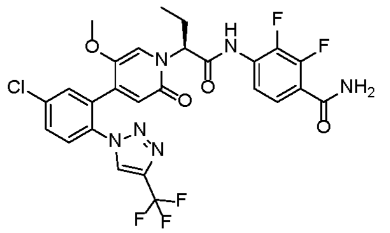 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2,3-difluorobenzamide |
| Compound 13 | 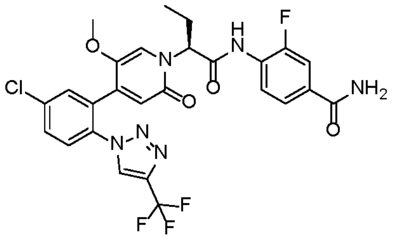 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-3-fluorobenzamide |
| Compound 14 | 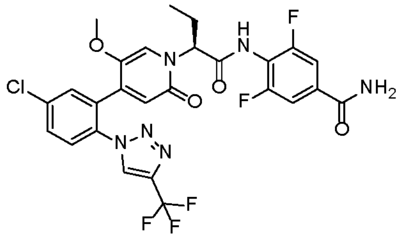 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-3,6-difluorobenzamide |
| Compound 15 | 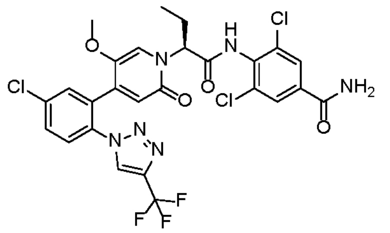 | (S)-3,5-dichloro-4-(2-(4-(5-chloro-2-(4-(trifluoromethyl)-1H-1,2,3-triazol-1-yl)phenyl)-5-methoxy-2-oxopyridin-1(2H)-yl)butanamido)benzamide |
| Compound 16 | 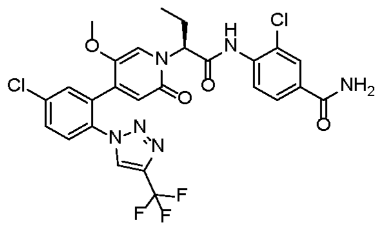 | (S)-3-chloro-4-(2-(4-(5-chloro-2-(4-(trifluoromethyl)-1H-1,2,3-triazol-1-yl)phenyl)-5-methoxy-2-oxopyridin-1(2H)-yl)butanamido)-benzamide |
| Compound 17 | 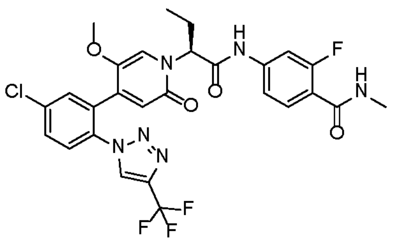 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2-fluoro-N-methylbenzamide |

(continued)

| No. | Structure | Name |
|---|---|---|
| Compound 18 | 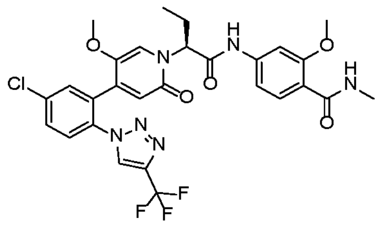 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2-methoxy-N-methylbenzamide |
| Compound 19 | 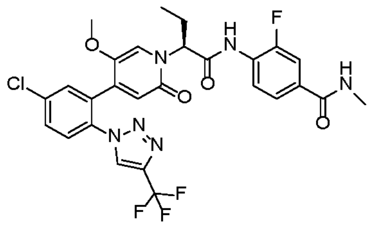 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-3-fluoro-N-methylbenzamide |
| Compound 20 | 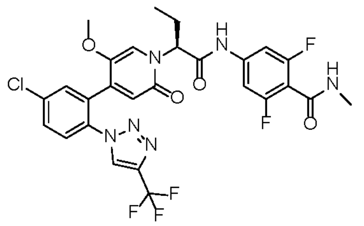 | (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2,6-difluoro-N-methylbenzamide |
| Compound 21 | 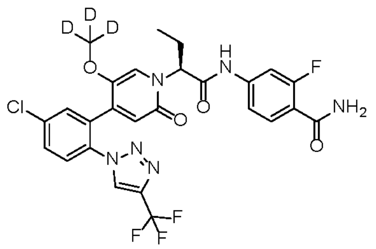 | (S)-4-(2-(4-(5-chloro-2-(4-(trifluoromethyl)-1H-1,2,3-triazol-1-yl)phenyl)-5-(methoxy-*d*$_3$)-2-oxopyridin-1(2H)-yl)butanamido)-2-fluorobenzamide |
| Compound 22 | 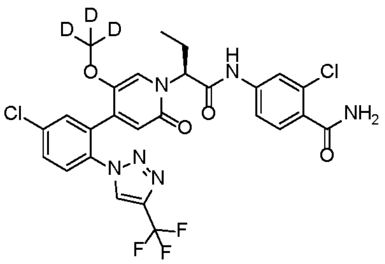 | (S)-2-chloro-4-(2-(4-(5-chloro-2-(4-(trifluoromethyl)-1H-1,2,3-triazol-1-yl)phenyl)-5-(methoxy-*d*$_3$)-2-oxopyridin-1(2H)-yl)butanamido)benzamide |
| Compound 23 | 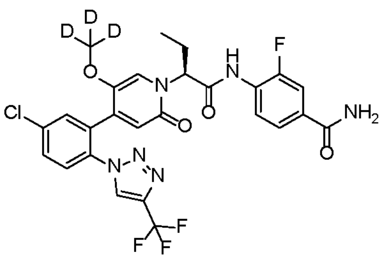 | (S)-4-(2-(4-(5-chloro-2-(4-(trifluoromethyl)-1H-1,2,3-triazol-1-yl)phenyl)-5-(methoxy-*d*$_3$)-2-oxopyridin-1(2H)-yl)butanamide)-3-fluorobenzamide |

(continued)

| No. | Structure | Name |
|---|---|---|
| Compound 24 | 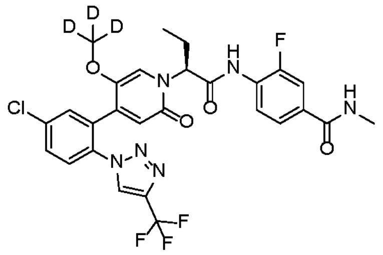 | (S)-4-(2-(4-(5-chloro-2-(4-(trifluoromethyl)-1H-1,2,3-triazol-1-yl)phenyl)-5-(methoxy-$d_3$)-2-oxopyridin-1(2H)-yl)butanamido)-3-fluoro-N-methylbenzamide |
| Compound 25 | 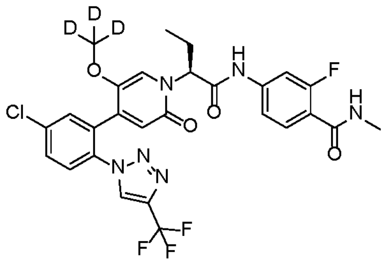 | (S)-4-(2-(4-(5-chloro-2-(4-(trifluoromethyl)-1H-1,2,3-triazol-1-yl)phenyl)-5-(methoxy-d3)-2-oxopyridin-1(2H)-yl)butanamido)-2-fluoro-N-methylbenzamide |
| Compound 26 | 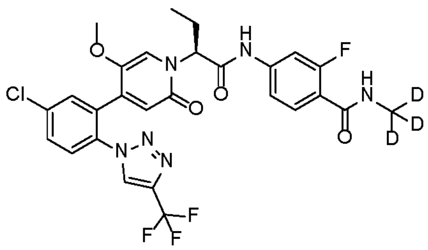 | (S)-4-(2-(4-(5-chloro-2-(4-(trifluoromethyl)-1H-1,2,3-triazol-1-yl)phenyl)-5-(methoxy)-2-oxopyridin-1(2H)-yl)butanamido)-2-fluoro-N-(methyl-$d_3$)benzamide |
| Compound 27 | 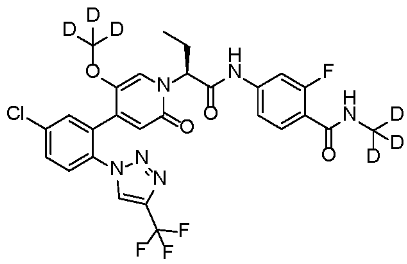 | (S)-4-(2-(4-(5-chloro-2-(4-(trifluoromethyl)-1H-1,2,3-triazol-1-yl)phenyl)-5-(methoxy-$d_3$)-2-oxopyridin-1(2H)-yl)butanamido)-2-fluoro-N-(methyl-$d_3$)benzamide |
| Compound 28 | 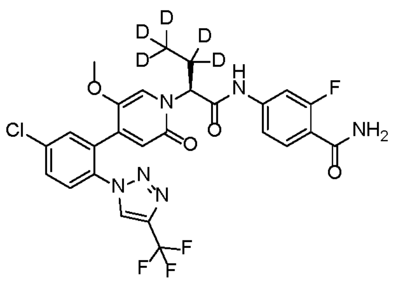 | (S)-4-(2-(4-(5-chloro-2-(4-(trifluoromethyl)-1H-1,2,3-triazol-1-yl)phenyl)-5-(methoxy)-2-oxopyridin-1(2H)-yl)butanamido-3,3,4,4,4-$d_5$)-2-fluorobenzamide |
| Compound 29 | 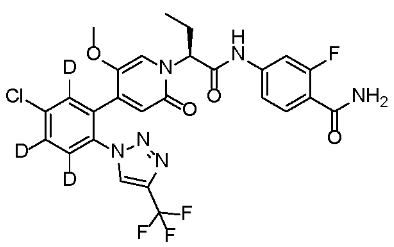 | (S)-4-(2-(4-(5-chloro-2-(4-(trifluoromethyl)-1H-1,2,3-triazol-1-yl)phenyl-3,4,6-$d_3$)-5-(methoxy)-2-oxopyridin-1(2H)-yl)butanamido)-2-fluorobenzamide |

(continued)

| No. | Structure | Name |
|---|---|---|
| Compound 30 | 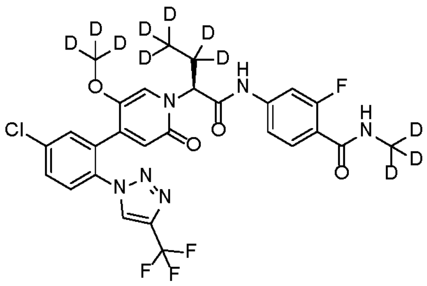 | (S)-4-(2-(4-(5-chloro-2-(4-(trifluoromethyl)-1H-1,2,3-triazol-1-yl)phenyl)-5-(methoxy-$d_3$)-2-oxopyridin-1(2H)-yl) butanamido-3,3,4,4,4-$d_5$)-2-fluoro-N-(methyl-$d_3$)benzamide |

In the present disclosure, if the chemical nomenclature conflicts with the structural formula, the compound structure represented by the numbering will control.

Further, hydrogen in the structure of the above compound, or the stereoisomer or pharmaceutically acceptable salt thereof may be substituted with one or more deuterium.

Further, the deuterated compound obtained by substituting the hydrogen in the structure of the compound above, or the stereoisomer or pharmaceutically acceptable salt thereof with one or more deuterium includes, but is not limited to, compound 28, compound 29 and compound 30.

In another aspect, the present disclosure provides a method for preparing the compound above, or the stereoisomer or pharmaceutically acceptable salt thereof, which includes the following steps:

when $R^2$ is $NH_2$, $NHR^7$ or $OR^8$, the synthesis route is as follows:

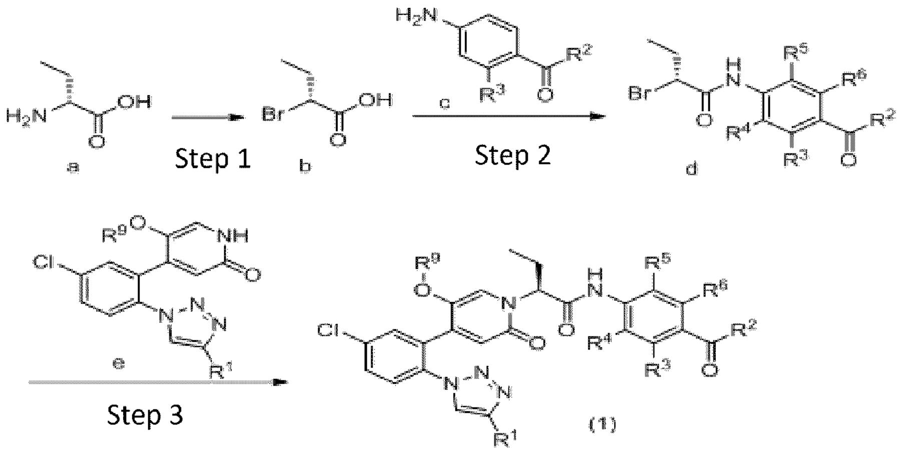


wherein $R^1$, $R^3$, $R^4$, $R^5$, $R^6$, $R^7$, $R^8$ and $R^9$ are defined correspondingly the same as in any one of claims 1-6;
step 1: bromination of starting material a to produce intermediate b;
step 2: condensation of intermediate b with starting material c to produce intermediate c; and step 3: a substitution reaction of intermediate d with compound e to produce a compound of formula (1); and
when $R^2$ is OH, the synthesis route is as follows:

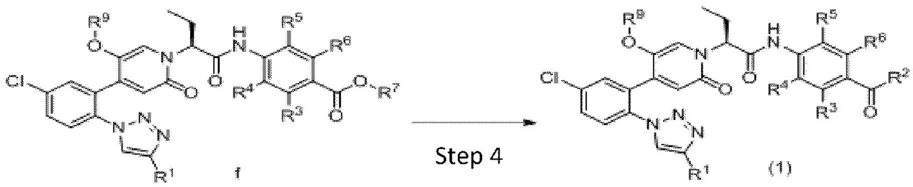


wherein $R^1$, $R^3$, $R^4$, $R^5$, $R^6$, $R^7$ and $R^9$ are defined correspondingly the same as in any one of claims 1-6;
step 4: hydrolysis of compound f under a basic condition to produce the compound of formula (1).

Further, the present disclosure further provides a key intermediate having a structure as represented by formula e

below:

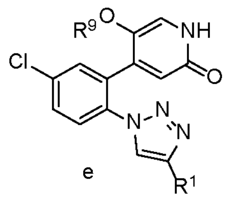

e

,

where $R^1$ is selected from halogen or trifluoromethyl, and $R^9$ is selected from $CH_3$ or $CD_3$; preferably, the $R^1$ is selected from halogen or trifluoromethyl, and $R^9$ is selected from $CD_3$.

Further, the intermediate described above is selected from the following compounds:

5-(5-chloro-2-(4-(trifluoromethyl)-1H-1,2,3-triazol-1-yl)phenyl)-5-(methoxy-d3)pyridine-2(1H)-one; and
4-(5-chloro-2-(4-chloro-1H-1,2,3-triazol-1-yl)phenyl)-5-(methoxy-d3)pyridine-2(1H)-one.

The aforementioned term "halogen" includes fluorine, chlorine, bromine or iodine; preferably fluorine, chlorine or bromine.

In a third aspect, the present disclosure provides a pharmaceutical composition comprising any one of the compounds described above, or the stereoisomer or pharmaceutically acceptable salt thereof, wherein the pharmaceutical composition further comprises a pharmaceutically acceptable excipient.

In a fourth aspect, the present disclosure further provides the use of the compound above, or the stereoisomer or pharmaceutically acceptable salt thereof in the preparation of drugs for treating and/or preventing an FXIa receptor-associated disease.

Further, the aforementioned FXIa receptor-associated disease is selected from a cerebrovascular arterial disease and/or a peripheral arterial disease.

Further, the aforementioned FXIa receptor-associated disease is selected from transient ischemic attack (TIA), ischemic stroke, including cardioembolic stroke such as stroke caused by atrial fibrillation, non-cardioembolic stroke such as lacunar stroke, stroke caused by an aortic or arteriolar disease or stroke of undetermined etiology, cryptogenic stroke, embolic stroke, embolic stroke of undetermined source, or an event leading to stroke or TIA and originating from thrombosis and/or thromboembolism, and/or a peripheral arterial condition leading to the peripheral arterial disease, including peripheral arterial occlusion, acute limb ischemia, amputation, reocclusion and restenosis after intervention (for example, angioplasty, stent implantation or surgery and bypass grafting), and/or stent thrombosis.

In the present disclosure, the term "alkyl" denotes a linear or branched saturated C1-C16 alkyl group. Other alkyl moieties that contain an "alkyl" substituent are explained in the same manner.

Beneficial effects: compared with the prior art, the compound of the present disclosure has a better in-vitro anticoagulant effect while exhibiting a highly consistent correlation with the in-vitro FXIa inhibitory effect. Additionally, the compounds in some examples of the present disclosure have superior pharmacokinetic characteristics in rats following injection administration. For example, compound 21, compound 25, compound 26, and compound 27 have better AUC and Cmax, thus having better efficacy. Furthermore, the compounds also have rapid elimination characteristics and higher safety.

## DETAILED DESCRIPTION OF THE INVENTION

The present disclosure will be further described below in details with reference to examples and test examples, which are intended to illustrate the technical solutions of the present disclosure only and should not be construed as limiting the present disclosure, and any equivalent substitution in the art that are made in accordance with the description of the present disclosure shall fall within the protection scope of the present disclosure.

All of the compounds, and the stereoisomer or pharmaceutically acceptable salt thereof in the present disclosure can be prepared by selecting the synthesis routes in the examples, and the conventional conditions of the reaction raw materials and reaction solvents can be adjusted according to the requirements for substituents or salt formation, which can be achieved by those skilled in the art based on the description of the present disclosure. Furthermore, unless otherwise stated, the column chromatography of the present disclosure refers to silica gel column chromatography, and unless otherwise stated, the elution solvent, whether it is a single or mixed elution solvent, can be determined based on the reaction solvent and the common general knowledge of those skilled in the art or using commonly used means.

The structure of the compound is determined by nuclear magnetic resonance ($^1H$ NMR) or liquid chromatography-mass spectrometry (LC-MS).

The liquid chromatography-mass spectrometry (LC-MS) instrument is Agilent G6120B (used with liquid-phase Agilent 1260). The nuclear magnetic resonator ($^1H$ NMR) is Bruker AVANCE-400 or Bruker AVANCE-800, the shift ($\delta$) of nuclear magnetic resonance ($^1H$ NMR) is given in parts per million (ppm), the solvent for determination is DMSO, the internal standard is tetramethylsilane (TMS), and the chemical shift is given in $10^{-6}$ (ppm).

The term "room temperature" in the present disclosure means a temperature in a range of 10-30°C.

**Example 1:** preparation of compound 1

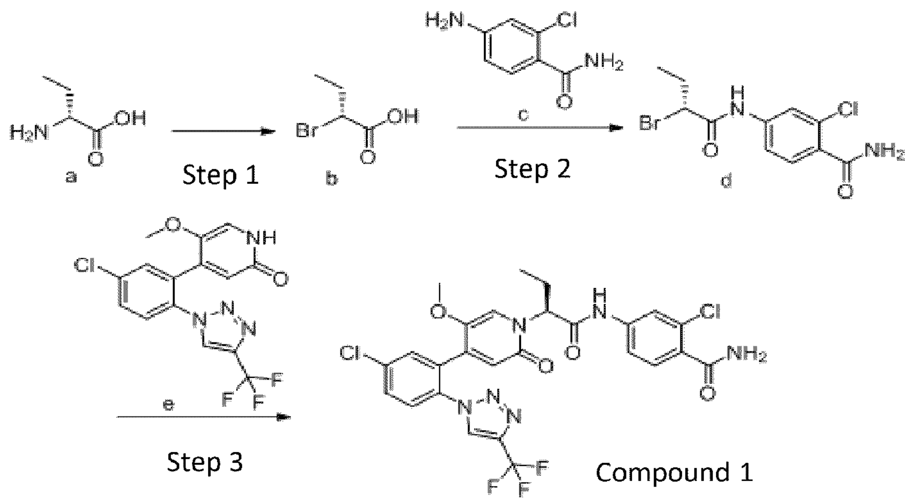


Step 1: (R)-2-bromo-3-propionic acid

5 g (48.49 mmol) of concentrated sulfuric acid was added to 60 ml of water and cooled to 5°C or lower, 23 g (193.3 mmol) of potassium bromide and 5 g (48.49 mmol) of D-2-aminobutyric acid were added, and 5 g (72.46 mmol) of sodium nitrite dissolved in 30 ml of water was added dropwise over about 30 min, with the temperature controlled at 0-5°C or lower. After the completion of addition, the mixture was stirred for reaction at 0-5°C overnight. On the next day, the reaction solution was extracted twice with ethyl acetate, the organic phases were combined, successively washed with water and saturated brine, and dried over anhydrous sodium sulfate, and the solvent was evaporated to dryness to obtain 7.5 g of an oil, which was directly used in the next step without purification.

Step 2: preparation of (R)-4-(2-bromobutanamido)-2-chlorobenzamide

441 mg (2.64 mmol) of (R)-2-bromo-3-propionic acid was dissolved in 4 ml of tetrahydrofuran, 300 mg (1.76 mmol) of 4-amino-2-chlorobenzamide was added, and the mixture was cooled to 0°C or lower; 654 mg (8.27 mmol) of pyridine was added, and then 2.24 g (3.52 mmol) of 1-propylphosphoric anhydride (50% ethyl acetate solution) diluted with 2 ml of tetrahydrofuran was added dropwise; and after the completion of addition, the solution was stirred at 0-5°C for 10 min and stirred at room temperature to allow a reaction for 30 min. After the reaction was completed, water was added to quench the reaction, and EA was added for extraction; the organic phase was successively washed with 5% citric acid, saturated sodium bicarbonate, water, and saturated brine, and dried over anhydrous sodium sulfate; and the solvent was evaporated to dryness to obtain 540 mg of a crude product. 5 ml of ethyl acetate was added to the crude product, and the mixture was stirred at room temperature for 2 h and filtered; and the filter cake was washed with ethyl acetate and dried under vacuum to obtain 430 mg of a white solid, with a yield of 76.4% and a purity of 96.55%.

ESI-MS: m/z = 319.0 $(M+H)^+$.

Step 3: preparation of title compound 1

200 mg (0.539 mmol) of 4-(5-chloro-2-(4-(trifluoromethyl)-1H-1,2,3-triazol-1-yl)phenyl)-5-methoxypyridine-2(1H)-one, 5 ml of isopropanol and 2 ml of acetone were mixed, 187 mg (1.624 mmol) of tetramethylguanidine was added and stirred for 5 min, and 207 mg (0.648 mmol) of (R)-4-(2-bromobutanamido)-2-chlorobenzamide was added, and stirred for reaction at room temperature overnight. After the reaction was completed, saturated ammonium chloride was added to quench the reaction, and ethyl acetate was added for extraction; the organic phase was successively

washed with water and saturated brine, and dried over anhydrous sodium sulfate; and the solvent was evaporated to dryness to obtain 400 mg of a crude product. The crude product was purified on a column with an eluent (ethyl acetate : petroleum ether = 1:2), and the product was collected to obtain 245 mg of a white solid. The yield was 74.6%, and the purity was 96.20%.

ESI-MS: m/z = 609.1 $(M+H)^+$.

$^1$H NMR (400 MHz, DMSO-d6) δ:10.67 (s, 1H), 9.14 (d, 1H), 7.98 - 7.72 (m, 5H), 7.60 - 7.30 (m, 3H), 7.13 (s, 1H), 6.53 (s, 1H), 5.51 (dd 1H), 3.25 (s, 3H), 2.14-2.04 (m, 2H), 0.78 (t, 3H).

**Example 2:** preparation of compound 2

Step 1: preparation of methyl (S)-2-chloro-4-(2-(4-(5-chloro-2-(4-(trifluoromethyl)-1H-1,2,3-triazol-1-yl)phenyl)-5-methoxy-2-oxopyridin-1(2H)-yl)butanamido)benzoate

The preparation method was the same as that in Example 1, except that 4-amino-2-chlorobenzamide in step 2 was replaced with methyl 4-amino-2-chlorobenzoate, to prepare the title compound, with a purity of 96.20%.

ESI-MS: m/z = 624.1 $(M+H)^+$.

Step 2: preparation of title compound 2

To 250 mg (0.40 mmol) of methyl (S)-2-chloro-4-(2-(4-(5-chloro-2-(4-(trifluoromethyl)-1H-1,2,3-triazol-1-yl) phenyl)-5-methoxy-2-oxopyridin-1(2H)-yl)butanamido)benzoate was added 5 ml of methanol, the mixture was heated to 50°C, 51 mg (1.215 mmol) of lithium hydroxide monohydrate dissolved in 2.5 ml of water was added, and the resulting solution was stirred for reaction at 50°C for 1 h. After the reaction was completed, 5% aqueous citric acid solution was added to quench the reaction, and ethyl acetate was added for extraction; the organic phase was successively washed with water and saturated brine, and dried over anhydrous sodium sulfate; and the solvent was evaporated to dryness to obtain 245 mg of a crude product. 5 ml of ethyl acetate was added to the crude product, and the mixture was stirred at room temperature for 2 h and filtered; and the filter cake was washed with ethyl acetate and dried under vacuum to obtain 200 mg of a white solid, with a yield of 81.9% and a purity of 97.89%.

ESI-MS: m/z = 610.1 $(M+H)^+$.

$^1$H NMR (400 MHz, DMSO-d6) δ:12.88 (s, 1H), 10.89 (s, 1H), 9.19 (s, 1H), 8.05 - 7.88 (m, 3H), 7.69 - 7.36 (m, 3H), 7.28 (s, 1H), 6.55 (s, 1H), 5.58 (dd 1H),3.28 (s, 3H), 2.15-2.03 (m, 2H), 0.76 (t, 3H).

**Example 3:** preparation of compound 3

The preparation method was the same as that in Example 1, except that 4-amino-2-chlorobenzamide in step 2 was replaced with 4-amino-2-chloro-N-methylbenzamide, to prepare the title compound 3, with a purity of 96.80%.

ESI-MS: m/z = 623.1 $(M+H)^+$.

$^1$H NMR (400 MHz, DMSO-d6) δ:10.58 (s, 1H), 9.11 (s, 1H), 7.91 - 7.66 (m, 4H), 7.58 - 7.25 (m, 3H), 7.15 (s, 1H), 6.51 (s, 1H), 5.53 (dd 1H), 3.26 (s, 3H), 2.88 (d, 3H),2.12-2.01 (m, 2H), 0.79 (t, 3H).

**Example 4:** preparation of compound 4

The preparation method was the same as that in Example 1, except that 4-amino-2-chlorobenzamide in step 2 was replaced with methyl 4-amino-2-chlorobenzoate, to prepare the title compound 4, with a purity of 97.90%.

ESI-MS: m/z = 624.1 $(M+H)^+$.

$^1$H NMR (400 MHz, DMSO-d6) δ:10.53 (s, 1H), 9.18 (s, 1H), 7.91 - 7.75 (m, 3H), 7.56 - 7.25 (m, 3H), 7.16 (s, 1H), 6.50 (s, 1H), 5.56 (dd 1H), 4.10 (s, 3H),3.28 (s, 3H), 2.18-2.07 (m, 2H), 0.79 (t, 3H).

**Example 5:** preparation of compound 5

The preparation method was the same as that in Example 1, except that 4-amino-2-chlorobenzamide in step 2 was replaced with 4-amino-2-methoxy-benzamide, to prepare the title compound 5, with a purity of 97.95.

ESI-MS: m/z = 605.2 $(M+H)^+$.

$^1$H NMR (400 MHz, DMSO-d6) δ:10.68 (s, 1H), 9.14 (d, 1H), 7.95 - 7.73 (m, 4H), 7.56 (m, 2H), 7.46 (d, 1H), 7.19 (dd, 1H), 7.14 (s, 1H), 6.53 (s, 1H), 5.54 (dd, 1H), 3.86 (s, 3H), 3.25 (s, 3H), 2.16-2.04 (m, 2H), 0.78 (t, 3H).

**Example 6:** preparation of compound 6

Step 1: preparation of methyl (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2-methoxybenzoate

The preparation method was the same as that in Example 1, except that 4-amino-2-chlorobenzamide in step 2 was replaced with methyl 4-amino-2-methoxy-benzoate, to prepare the title compound, with a purity of 97.20%. ESI-MS: m/z = 620.0 $(M+H)^+$.

Step 2: preparation of title compound 6

To 250 mg (0.403 mmol) of methyl (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2-methoxybenzoate was added 6 ml of methanol, 68 mg (1.62 mmol) of lithium hydroxide monohydrate dissolved in 3 ml of water was added, and the mixture was stirred for reaction at 60°C for 1 h. After the reaction was completed, the system was cooled to room temperature, 5% aqueous citric acid solution was added to quench the reaction, and ethyl acetate was added for extraction; the organic phase was successively washed with water and saturated brine, and dried over anhydrous sodium sulfate; and the solvent was evaporated to dryness to obtain 250 mg of a crude product. The crude product was purified using a column with an eluent (dichloromethane : methanol = 50 : 1), and the target product was collected to obtain 198 mg of a white solid, with a yield of 81.1% and a purity of 97.66%.

ESI-MS: m/z = 606.1 $(M+H)^+$.

$^1H$ NMR (400 MHz, DMSO-d6) δ:12.35 (s, 1H), 10.68 (s, 1H), 9.14 (d, 1H), 7.93 - 7.75 (m, 3H), 7.68 (d, 1H), 7.54 (d, 1H), 7.22 - 7.10 (m, 2H), 6.54 (s, 1H), 5.54 (dd, 1H), 3.78 (s, 3H), 3.25 (s, 3H), 2.14-2.04 (m, 2H), 0.79 (t, 3H).

**Example 7:** preparation of compound 7

The preparation method was the same as that in Example 1, except that 4-amino-2-chlorobenzamide in step 2 was replaced with 4-amino-2-trifluoromethyl-benzamide, to prepare the title compound 7, with a purity of 95.21%.

ESI-MS: m/z = 643.1 $(M+H)^+$.

$^1H$ NMR (400 MHz, DMSO-d6) δ:10.80 (s, 1H), 9.13 (s, 1H), 8.12 (d, 1H), 8.02 - 7.72 (m, 5H), 7.54-7.51 (m, 2H), 7.14 (s, 1H), 6.54 (s, 1H), 5.51 (dd, 1H), 3.25 (s, 3H), 2.23 - 2.03 (m, 2H), 0.79 (t, 3H).

**Example 8:** preparation of compound 8

The preparation method was the same as that in Example 1, except that 4-amino-2-chlorobenzamide in step 2 was replaced with 4-amino-1,2-dibenzamide, to prepare the title compound 8, with a purity of 98.69%.

ESI-MS: m/z = 618.2 $(M+H)^+$.

$^1H$ NMR (400 MHz, DMSO-d6) δ:11.09 (s, 1H), 9.20 (s, 1H), 8.28 - 7.96 (m, 5H), 7.85 - 7.59 (m, 5H), 7.28 (s, 1H), 6.96 (s, 1H), 5.98 (dd 1H), 3.29 (s, 3H), 2.23-2.15 (m, 2H), 0.80 (t, 3H).

**Example 9:** preparation of compound 9

The preparation method was the same as that in Example 2, except that 4-(5-chloro-2-(4-(trifluoromethyl)-1H-1,2,3-triazol-1-yl)phenyl)-5-methoxypyridine-2(1H)-one was replaced with 4-(5-chloro-2-(4-chloro-1H-1,2,3-triazol-1-yl)phenyl)-5-methoxypyridine-2(1H)-one, to prepare the title compound 9, with a purity of 97.25%.

ESI-MS: m/z = 576.1 $(M+H)^+$.

$^1H$ NMR (400 MHz, DMSO-d6) δ:12.88 (s, 1H), 10.89 (s, 1H), 9.19 (s, 1H), 8.01 - 7.85 (m, 3H), 7.68 - 7.32 (m, 3H), 7.25 (s, 1H), 6.53 (s, 1H), 5.59 (dd 1H),3.27 (s, 3H), 2.16-2.02 (m, 2H), 0.77 (t, 3H).

**Example 10:** preparation of compound 10

The preparation method was the same as that in Example 1, except that 4-(5-chloro-2-(4-(trifluoromethyl)-1H-1,2,3-triazol-1-yl)phenyl)-5-methoxypyridine-2(1H)-one in step 3 was replaced with 4-(5-chloro-2-(4-chloro-1H-1,2,3-triazol-1-yl)phenyl)-5-methoxypyridine-2(1H)-one, to prepare the title compound 10, with a purity of 98.14%.

ESI-MS: m/z = 575.1 $(M+H)^+$.

$^1H$ NMR (400 MHz, DMSO-d6) δ:10.70 (s, 1H), 9.15 (d, 1H), 7.97 - 7.71 (m, 5H), 7.59 - 7.28 (m, 3H), 7.15 (s, 1H), 6.54 (s, 1H), 5.52 (dd 1H), 3.28 (s, 3H), 2.15-2.03 (m, 2H), 0.78 (t, 3H).

**Example 11:** preparation of compound 11

The preparation method was the same as that in Example 7, except that 4-amino-2-chlorobenzamide in step 2 was replaced with 4-amino-2-trifluoromethyl-benzamide, and then 4-(5-chloro-2-(4-(trifluoromethyl)-1H-1,2,3-triazol-1-yl)phenyl)-5-methoxypyridine-2(1H)-one in step 3 was replaced with 4-(5-chloro-2-(4-chloro-1H-1,2,3-triazol-1-yl)phenyl)-5-methyloxypyridine-2(1H)-one, to prepare the title compound 11, with a purity of 97.46%.

ESI-MS: m/z = 609.1 $(M+H)^+$.

$^1$H NMR (400 MHz, DMSO-d6) δ:10.79 (s, 1H), 9.11 (s, 1H), 8.10 (d, 1H), 8.00 - 7.69 (m, 5H), 7.56-7.53 (m, 2H), 7.18 (s, 1H), 6.56 (s, 1H), 5.53 (dd, 1H), 3.28 (s, 3H), 2.21 - 2.00 (m, 2H), 0.77 (t, 3H).

**Example 12:** preparation of compound 12

The preparation method was the same as that in Example 1, except that 4-amino-2-chlorobenzamide in step 2 was replaced with 4-amino-2,3-difluoro-benzamide, to prepare the title compound 12, with a purity of 98.27%.

ESI-MS: m/z = 611.1 $(M+H)^+$.

$^1$H NMR (400 MHz, DMSO-d6) δ: 10.57 (s, 1H), 9.15 (s, 1H), 7.88 - 7.81 (m, 2H), 7.79 (d, J = 2.1 Hz, 2H), 7.71 (d, J = 9.1 Hz, 2H), 7.53 - 7.37 (m, 1H), 7.10 (s, 1H), 6.55 (s, 1H), 5.68 (s, 1H), 3.24 (s, 3H), 2.11 (dt, J = 17.0, 8.6 Hz, 2H), 0.79 (t, J = 7.2 Hz, 3H).

**Example 13:** preparation of compound 13

The preparation method was the same as that in Example 1, except that 4-amino-2-chlorobenzamide in step 2 was replaced with 4-amino-3-fluoro-benzamide, to prepare the title compound 13, with a purity of 98.64%.

ESI-MS: m/z = 593.1 $(M+H)^+$.

$^1$H NMR (400 MHz, DMSO-d6) δ: 10.41 (s, 1H), 9.15 (d, J = 1.0 Hz, 1H), 8.05 - 7.93 (m, 2H), 7.88 - 7.81 (m, 2H), 7.80 - 7.78 (m, 1H), 7.78 - 7.67 (m, 2H), 7.47 (s, 1H), 7.11 (s, 1H), 6.54 (s, 1H), 5.71 (s, 1H), 3.24 (s, 3H), 2.25 - 1.95 (m, 2H), 0.78 (t, J = 7.2 Hz, 3H).

**Example 14:** preparation of compound 14

The preparation method was the same as that in Example 1, except that 4-amino-2-chlorobenzamide in step 2 was replaced with 4-amino-3,5-difluoro-benzamide, to prepare the title compound 14, with a purity of 98.76%.

ESI-MS: m/z = 611.1 $(M+H)^+$.

$^1$H NMR (400 MHz, DMSO-d6) δ: 10.37 (s, 1H), 9.13 (s, 1H), 8.13 (s, 1H), 7.87 - 7.80 (m, 2H), 7.78 (d, J = 2.0 Hz, 1H), 7.69 (s, 1H), 7.64 (d, J = 8.6 Hz, 2H), 7.07 (s, 1H), 6.56 (s, 1H), 5.65 (s, 1H), 3.22 (s, 3H), 2.15 - 1.95 (m, 2H), 0.81 (t, J = 7.2 Hz, 3H).

**Example 15:** preparation of compound 15

The preparation method was the same as that in Example 1, except that 4-amino-2-chlorobenzamide in step 2 was replaced with 4-amino-3,5-dichloro-benzamide, to prepare the title compound 15, with a purity of 98.12%.

ESI-MS: m/z = 643.1 $(M+H)^+$.

$^1$H NMR (400 MHz, DMSO-d6) δ: 10.37 (s, 1H), 9.13 (s, 1H), 8.13 (s, 1H), 7.92 - 7.88 (m, 2H), 7.82-7.78 (m, 1H), 7.69 (s, 1H), 7.66-7.62(m, 2H), 7.07 (s, 1H), 6.56 (s, 1H), 5.65 (s, 1H), 3.22 (s, 3H), 2.18 - 1.98 (m, 2H), 0.81 (t, J = 7.2 Hz, 3H).

**Example 16:** preparation of compound 16

The preparation method was the same as that in Example 1, except that 4-amino-2-chlorobenzamide in step 2 was replaced with 4-amino-3-chloro-benzamide, to prepare the title compound 16, with a purity of 98.48%.

ESI-MS: m/z = 609.1 $(M+H)^+$.

$^1$H NMR (400 MHz, DMSO-d6) δ:10.08 (s, 1H), 9.16 (s, 1H), 8.07 (s, 1H), 8.00 (s, 1H), 7.84 (m, 4H), 7.79 (d, J = 2.1 Hz, 1H), 7.50 (s, 1H), 7.10 (s, 1H), 6.57 (s, 1H), 5.69 (s, 1H), 3.24 (s, 3H), 2.28 - 2.03 (m, 2H), 0.80 (t, J = 7.2 Hz, 3H).

**Example 17:** preparation of compound 17

The preparation method was the same as that in Example 1, except that 4-amino-2-chlorobenzamide in step 2 was replaced with 4-amino-2-fluoro-N-methylbenzamide, to prepare the title compound 17, with a purity of 97.95%.

ESI-MS: m/z = 607.1 $(M+H)^+$.

$^1H$ NMR (400 MHz, DMSO-d6) δ: 10.83 (s, 1H), 9.14 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.25 (s, 3H), 2.76 (d, J = 4.6 Hz, 3H), 2.18 - 2.02 (m, 2H), 0.78 (t, J = 7.2 Hz, 3H).

**Example 18:** preparation of compound 18

The preparation method was the same as that in Example 1, except that 4-amino-2-chlorobenzamide in step 2 was replaced with 4-amino-2-methoxy-N-methylbenzamide, to prepare the title compound 18, with a purity of 96.25%.

ESI-MS: m/z = 619.2 $(M+H)^+$.

$^1H$ NMR (400 MHz, DMSO-d6) δ: 10.72 (s, 1H), 9.14 (d, J = 1.1 Hz, 1H), 8.13 - 8.06 (m, 1H), 7.89 - 7.80 (m, 2H), 7.79 (d, J = 2.0 Hz, 1H), 7.70 - 7.60 (m, 2H), 7.37 (dd, J = 8.5, 2.0 Hz, 1H), 7.13 (s, 1H), 6.54 (s, 1H), 5.52 (t, J = 7.8 Hz, 1H), 3.86 (s, 3H), 3.25 (s, 3H), 2.76 (d, J = 4.6 Hz, 3H), 2.18 - 2.02 (m, 2H), 0.78 (t, J = 7.2 Hz, 3H).

**Example 19:** preparation of compound 19

The preparation method was the same as that in Example 1, except that 4-amino-2-chlorobenzamide in step 2 was replaced with 4-amino-3-fluoro-N-methylbenzamide, to prepare the title compound 19, with a purity of 98.20%.

ESI-MS: m/z = 607.1 $(M+H)^+$.

$^1H$ NMR (400 MHz, DMSO-d6) δ:10.40 (s, 1H), 9.15 (d, J = 1.0 Hz, 1H), 8.49 (d, J = 4.6 Hz, 1H), 7.98 (t, J = 8.1 Hz, 1H), 7.89 - 7.80 (m, 2H), 7.79 (dd, J = 2.1, 0.7 Hz, 1H), 7.71 (dd, J = 11.8, 1.9 Hz, 1H), 7.66 (dd, J = 8.4, 2.0 Hz, 1H), 7.11 (s, 1H), 6.54 (s, 1H), 5.70 (s, 1H), 3.24 (s, 3H), 2.77 (d, J = 4.5 Hz, 3H), 2.12 (dt, J = 15.2, 7.5 Hz, 2H), 0.78 (t, J = 7.1 Hz, 3H).

**Example 20:** preparation of compound 20

The preparation method was the same as that in Example 1, except that 4-amino-2-chlorobenzamide in step 2 was replaced with 4-amino-2,5-difluoro-N-methylbenzamide, to prepare the title compound 20, with a purity of 98.20%.

ESI-MS: m/z = 625.1 $(M+H)^+$.

$^1H$ NMR (400 MHz, DMSO-d6) δ: 10.85 (s, 1H), 9.14 (d, J = 1.1 Hz, 1H), 8.55 (q, J = 4.6 Hz, 1H), 7.88 - 7.81 (m, 2H), 7.80 - 7.76 (m, 1H), 7.36 (d, J = 9.8 Hz, 2H), 7.12 (s, 1H), 6.54 (s, 1H), 5.48 (s, 1H), 3.25 (s, 3H), 2.75 (d, J = 4.6 Hz, 3H), 2.08 (tt, J = 16.0, 8.4 Hz, 2H), 0.78 (t, J = 7.2 Hz, 3H).

**Example 21:** preparation of compound 21

The preparation method was the same as that in Example 1, except that 4-amino-2-chlorobenzamide in step 2 was replaced with 4-amino-2-fluorobenzamide, and 4-(5-chloro-2-(4-(trifluoromethyl)-1H-1,2,3-triazol-1-yl)phenyl)-5-methoxypyridine-2(1H)-one in step 3 was replaced with 4-(5-chloro-2-(4-(trifluoromethyl)-1H-1,2,3-triazol-1-yl)phenyl)-5-(methoxy-d3)pyridine-2(1H)-one, to prepare the title compound 21, with a purity of 97.65%.

ESI-MS: m/z = 596.2 $(M+H)^+$.

$^1H$ NMR (400 MHz, DMSO-d6) δ: 10.79 (s, 1H), 9.14 (d, J = 1.0 Hz, 1H), 7.89 - 7.81 (m, 2H), 7.79 (dd, J = 2.1, 0.7 Hz, 1H), 7.74 - 7.60 (m, 2H), 7.55 (d, J = 11.3 Hz, 2H), 7.37 (dd, J = 8.6, 2.0 Hz, 1H), 7.13 (s, 1H), 6.54 (s, 1H), 5.53 (s, 1H), 2.16 - 2.05 (m, 2H), 0.78 (t, J = 7.2 Hz, 3H).

The structural confirmation data of the intermediate in step 3: 4-(5-chloro-2-(4-(trifluoromethyl)-1H-1,2,3-triazol-1-yl)phenyl)-5-(methoxy-d3)pyridine-2(1H)-one was as follows:

( 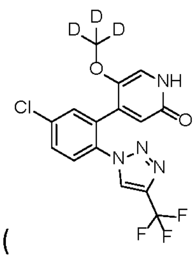 )

ESI-MS: m/z = 374.1 $(M+H)^+$.

$^1H$ NMR (400 MHz, DMSO-d6) δ: 1H NMR (400 MHz, DMSO-d6) δ: 11.21 (s, 1H), 9.18 (s, 1H), 7.81 (s, 2H), 7.72 (s, 1H), 695 (s, 1H), 6.41 (s, 1H).

**Example 22:** preparation of compound 22

The preparation method was the same as that in Example 1, except that 4-(5-chloro-2-(4-(trifluoromethyl)-1H-1,2,3-triazol-1-yl)phenyl)-5-methoxypyridine-2(1H)-one in step 3 was replaced with 4-(5-chloro-2-(4-(trifluoromethyl)-1H-1,2,3-triazol-1-yl)phenyl)-5-(methoxy-$d_3$)pyridine-2(1H)-one, to prepare the title compound 22, with a purity of 97.65%.
ESI-MS: m/z = 612.1 $(M+H)^+$.
$^1$H NMR (400 MHz, DMSO-d6) δ: 10.79 (s, 1H), 9.14 (d, J = 1.0 Hz, 1H), 7.97 - 7.79 (m, 2H), 7.79 (dd, J = 2.1, 0.7 Hz, 1H), 7.74 - 7.60 (m, 2H), 7.55 (d, J = 11.3 Hz, 2H), 7.37 (dd, J = 8.6, 2.0 Hz, 1H), 7.13 (s, 1H), 6.54 (s, 1H), 5.53 (s, 1H), 2.16 - 2.05 (m, 2H), 0.78 (t, J = 7.2 Hz, 3H).

**Example 23:** preparation of compound 23

The preparation method was the same as that in Example 1, except that 4-amino-2-chlorobenzamide in step 2 was replaced with 4-amino-3-fluorobenzamide, and 4-(5-chloro-2-(4-(trifluoromethyl)-1H-1,2,3-triazol-1-yl)phenyl)-5-methoxypyridine-2(1H)-one in step 3 was replaced with 4-(5-chloro-2-(4-(trifluoromethyl)-1H-1,2,3-triazol-1-yl)phenyl)-5-(methoxy-$d_3$)pyridine-2(1H)-one, to prepare the title compound 23, with a purity of 97.65%.
ESI-MS: m/z = 596.2 $(M+H)^+$.
$^1$H NMR (400 MHz, DMSO-d6) δ:10.41 (s, 1H), 9.15 (d, J = 1.0 Hz, 1H), 8.05 - 7.93 (m, 2H), 7.88 - 7.81 (m, 2H), 7.80 - 7.78 (m, 1H), 7.78 - 7.67 (m, 2H), 7.47 (s, 1H), 7.11 (s, 1H), 6.54 (s, 1H), 5.71 (s, 1H), 2.25 - 1.95 (m, 2H), 0.78 (t, J = 7.2 Hz, 3H).

**Example 24:** preparation of compound 24

The preparation method was the same as that in Example 1, except that 4-amino-2-chlorobenzamide in step 2 was replaced with 4-amino-3-fluoro-N-methylbenzamide, and 4-(5-chloro-2-(4-(trifluoromethyl)-1H-1,2,3-triazol-1-yl) phenyl)-5-methoxypyridine-2(1H)-one in step 3 was replaced with 4-(5-chloro-2-(4-(trifluoromethyl)-1H-1,2,3-triazol-1-yl)phenyl)-5-(methoxy-$d_3$)pyridine-2(1H)-one, to prepare the title compound 24, with a purity of 97.65%.
ESI-MS: m/z = 610.2 $(M+H)^+$.
$^1$H NMR (400 MHz, DMSO-d6) δ: 10.40 (s, 1H), 9.15 (d, J = 1.0 Hz, 1H), 8.49 (d, J = 4.6 Hz, 1H), 7.98 (t, J = 8.1 Hz, 1H), 7.89 - 7.80 (m, 2H), 7.79 (dd, J = 2.1, 0.7 Hz, 1H), 7.71 (dd, J = 11.8, 1.9 Hz, 1H), 7.66 (dd, J = 8.4, 2.0 Hz, 1H), 7.11 (s, 1H), 6.54 (s, 1H), 5.70 (s, 1H), 2.77 (d, J = 4.5 Hz, 3H), 2.12 (dt, J = 15.2, 7.5 Hz, 2H), 0.78 (t, J = 7.1 Hz, 3H).

**Example 25:** preparation of compound 25

The preparation method was the same as that in Example 1, except that 4-amino-2-chlorobenzamide in step 2 was replaced with 4-amino-2-fluoro-N-methylbenzamide, and 4-(5-chloro-2-(4-(trifluoromethyl)-1H-1,2,3-triazol-1-yl) phenyl)-5-methoxypyridine-2(1H)-one in step 3 was replaced with 4-(5-chloro-2-(4-(trifluoromethyl)-1H-1,2,3-triazol-1-yl)phenyl)-5-(methoxy-d3)pyridine-2(1H)-one, to prepare the title compound 25, with a purity of 98.85%.
ESI-MS: m/z = 610.2 $(M+H)^+$.
$^1$H NMR (400 MHz, DMSO-d6) δ: 10.80 (s, 1H), 9.20 - 9.08 (m, 1H), 8.10 (q, J = 4.2 Hz, 1H), 7.93 - 7.81 (m, 2H), 7.78 (d, J = 2.1 Hz, 1H), 7.70 - 7.58 (m, 2H), 7.37 (dd, J = 8.6, 2.0 Hz, 1H), 7.13 (s, 1H), 6.54 (s, 1H), 5.52 (s, 1H), 2.76 (d, J = 4.6 Hz, 3H), 2.20 - 2.02 (m, J = 7.6 Hz, 2H), 0.78 (t, J = 7.2 Hz, 3H).

**Example 26:** preparation of compound 26

The preparation method was the same as that in Example 1, except that 4-amino-2-chlorobenzamide in step 2 was replaced with 4-amino-2-fluoro-N-(methyl-d3)benzamide, to prepare the title compound 26, with a purity of 99.12%.
ESI-MS: m/z = 610.2 $(M+H)^+$.
$^1$H NMR (400 MHz, DMSO-d6) δ:10.79 (s, 1H), 9.14 (d, J = 1.1 Hz, 1H), 8.06 (d, J = 3.4 Hz, 1H), 7.92 - 7.81 (m, 2H), 7.81 - 7.76 (m, 1H), 7.70 - 7.60 (m, 2H), 7.37 (dd, J = 8.6, 2.0 Hz, 1H), 7.13 (s, 1H), 6.54 (s, 1H), 5.51 (d, J = 8.6 Hz, 1H), 3.25 (s, 3H), 2.19 - 1.99 (m, J = 7.1 Hz, 2H), 0.78 (t, J = 7.2 Hz, 3H).

**Example 27:** preparation of compound 27

The preparation method was the same as that in Example 1, except that 4-amino-2-chlorobenzamide in step 2 was replaced with 4-amino-2-fluoro-N-(methyl-d3)benzamide, and 4-(5-chloro-2-(4-(trifluoromethyl)-1H-1,2,3-triazol-1-yl)phenyl)-5-methoxypyridine-2(1H)-one in step 3 was replaced with 4-(5-chloro-2-(4-(trifluoromethyl)-1H-1,2,3-triazol-1-yl)phenyl)-5-(methoxy-d3)pyridine-2(1H)-one, to prepare the title compound 27, with a purity of 98.07%.

ESI-MS: m/z = 613.2 $(M+H)^+$.

$^1H$ NMR (400 MHz, DMSO-d6) δ: 10.79 (s, 1H), 9.14 (d, J = 1.1 Hz, 1H), 8.06 (d, J = 3.4 Hz, 1H), 7.90 - 7.76 (m, 3H), 7.71 - 7.60 (m, 2H), 7.37 (dd, J = 8.6, 2.0 Hz, 1H), 7.13 (s, 1H), 6.54 (s, 1H), 5.52 (s, 1H), 2.09 (h, J = 7.1 Hz, 2H), 0.78 (t, J = 7.2 Hz, 3H).

**Example 28:** preparation of compound 28

The preparation method was the same as that in Example 1, except that D-2-aminobutyric acid in step 1 was replaced with (R)-2-aminobutyric acid-3,3,4,4,4-d5 acid, and 4-amino-2-chlorobenzamide in step 2 was replaced with 4-amino-2-fluoro-benzamide, to prepare the title compound 28, with a purity of 97.36%.

ESI-MS: m/z = 598.2 $(M+H)^+$.

$^1H$ NMR (400 MHz, DMSO-d6) δ: 10.72 (s, 1H), 9.10 (d, 1H), 7.98 - 7.74 (m, 5H), 7.62 - 7.36 (m, 3H), 7.11 (s, 1H), 6.56 (s, 1H), 5.50 (dd 1H), 3.26 (s, 3H).

**Example 29:** preparation of compound 29

The preparation method was the same as that in Example 1, except that 4-amino-2-chlorobenzamide in step 2 was replaced with 4-amino-2-fluoro-benzamide, and 4-(5-chloro-2-(4-(trifluoromethyl)-1H-1,2,3-triazol-1-yl)phenyl)-5-methoxypyridine-2(1H)-one in step 3 was replaced with 4-(5-chloro-2-(4-(trifluoromethyl)-1H-1,2,3-triazol-1-yl)phenyl-3,4,6-d3)-5-(methyloxy)pyridine-2(1H)-one, to prepare the title compound 29, with a purity of 97.66%.

ESI-MS: m/z = 596.2 $(M+H)^+$.

$^1H$ NMR (400 MHz, DMSO-d6)δ: 10.80 (s, 1H), 9.15 (d, 1H), 7.92 - 7.82 (m, 2H), 7.77 (d, J = 2.1 Hz, 1H), 7.72 - 7.62 (m, 2H), 7.13 (s, 1H), 6.54 (s, 1H), 5.52 (s, 1H), 3.28 (s, 3H), 2.21 - 2.03 (m, J = 7.6 Hz, 2H), 0.79 (t, J = 7.2 Hz, 3H).

**Example 30:** preparation of compound 30

The preparation method was the same as that in Example 1, except that D-2-aminobutyric acid in step 1 was replaced with (R)-2-aminobutyric acid-3,3,4,4,4-d5 acid, 4-amino-2-chlorobenzamide in step 2 was replaced with 4-amino-2-fluoro-N-(methyl-d3)benzamide, and 4-(5-chloro-2-(4-(trifluoromethyl)-1H-1,2,3-triazol-1-yl)phenyl)-5-methoxypyridine-2(1H)-one in step 3 was replaced with 4-(5-chloro-2-(4-(trifluoromethyl)-1H-1,2,3-triazol-1-yl)phenyl)-5-(methoxy-d3)pyridine-2(1H)-one, to prepare the title compound 30, with a purity of 98.62%.

ESI-MS: m/z = 618.2 $(M+H)^+$.

$^1H$ NMR (400 MHz, DMSO-d6) δ:10.78(s, 1H), 9.16 (d, J = 1.1 Hz, 1H), 8.06 (d, J = 3.4 Hz, 1H), 7.92 - 7.78 (m, 3H), 7.71 - 7.60 (m, 2H), 7.37 (dd, J = 8.6, 2.0 Hz, 1H), 7.13 (s, 1H), 6.54 (s, 1H), 5.52 (s, 1H).

**Comparative example 1:** preparation of (S)-4-(2-(4-(5-chloro-2-(4-(trifluoromethyl)-1H-1,2,3-triazol-1-yl)phenyl)-5-methoxy-2-oxopyridin-1(2H)-yl)butanamido)-2-fluorobenzamide

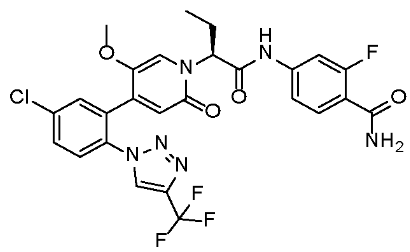

The compound was synthesized following the method described in patent CN 108026072 B, with a purity of 98.5%.

ESI-MS: *m/z* = 593.1 $(M+H)^+$.

$^1H$ NMR (400 MHz, DMSO-d6) δ: 10.78 (s, 1H), 9.14 (s, 1H), 7.88 - 7.77 (m, 3H), 7.72 - 7.61 (m, 2H), 7.55 (d, 2H), 7.37 (dd, 1H), 7.13 (s, 1H), 6.54 (s, 1H), 5.52 (dd, 1H), 3.25 (s, 3H), 2.18 - 2.00 (m, 2H), 0.78 (t, 3H).

**Test example 1: determination of in-vitro anticoagulant effect in human plasma**

### 1. Test sample

Compounds 1-30 in examples and compound in comparative example 1.

### 2. Test method

The blood of a healthy human was collected using a sodium citrate anticoagulant tube (1 : 9), immediately mixed with the anticoagulant until uniform, and centrifuged at 4,000 r/min at room temperature for 15 min. After centrifugation, the plasma sample was pipetted and frozen (-80°C) for later use.

A proper amount of a compound was weighed and prepared into a 10-100 mM stock solution using 100% of DMSO (the specific concentration of the stock solution was determined based on the properties of the compound); and then working solutions with various concentrations (specifically: 1 μM, 3 μM and 10 μM, respectively) were prepared from the stock solution using the healthy human plasma as a solution and thoroughly mixed. After incubation at 37°C for 3 min, the sample was placed on an instrument (model: CS-2000I) to determine APTT.

### 3. Data processing

GraphPad Prism software was used for analysis to calculate the APTT prolongation rate at each concentration.

### 4. Test results

The results are shown in the table below. As shown, the APTT prolongation rates for all tested compounds 1, 2, 5, 6, 13, 17, 21, 25, 26 and 27 at 1 μM were superior to that of the compound in comparative example 1 and are greater than 50%. Based on the test results, it can be estimated that the EC150 of the aforementioned compounds of the present disclosure is less than 1 μM.

**Table 1 Test results of in-vitro anticoagulant effect in human plasma**

| Sample | APTT prolongation rate (%) | | |
|---|---|---|---|
| | 1 μM | 3 μM | 10 μM |
| Compound 1 | 55.9 | 65.8 | 136.5 |
| Compound 2 | 63.5 | 88.9 | 124.8 |
| Compound 3 | 48.2 | 66.5 | 92.0 |
| Compound 4 | 31.5 | 54.3 | 102.6 |
| Compound 5 | 56.0 | 84.6 | 125.8 |
| Compound 6 | 66.3 | 78.0 | 135.1 |
| Compound 7 | 37.9 | 52.4 | 73.4 |
| Compound 8 | 34.7 | 64.5 | 93.8 |
| Compound 9 | 44.3 | 69.5 | 103.8 |
| Compound 10 | 38.6 | 56.0 | 98.9 |
| Compound 11 | 18.7 | 26.9 | 42.0 |
| Compound 12 | 23.2 | 33.5 | 81.1 |
| Compound 13 | 50.2 | 80.2 | 175.9 |
| Compound 14 | 0.9 | 9.1 | 18.5 |
| Compound 15 | 24.2 | 30.0 | 51.1 |
| Compound 16 | 7.0 | 11.7 | 26.8 |
| Compound 17 | 57.7 | 82.6 | 145.3 |
| Compound 18 | 33.2 | 46.1 | 90.6 |
| Compound 19 | 24.2 | 44.0 | 77.1 |
| Compound 20 | 19.2 | 49.2 | 98.6 |

(continued)

| Sample | APTT prolongation rate (%) | | |
|---|---|---|---|
| | 1 μM | 3 μM | 10 μM |
| Compound 21 | 52.3 | 76.8 | 147.6 |
| Compound 22 | 35.6 | 62.0 | 105.9 |
| Compound 23 | 48.8 | 81.2 | 168.0 |
| Compound 24 | 35.1 | 73.2 | 132.5 |
| Compound 25 | 56.8 | 76.7 | 158.5 |
| Compound 26 | 53.2 | 78.3 | 159.5 |
| Compound 27 | 55.8 | 79.7 | 142.5 |
| Compound 28 | 35.8 | 63.3 | 102.9 |
| Compound 29 | 38.9 | 73.1 | 105.5 |
| Compound 30 | 48.1 | 79.0 | 138.4 |
| Comparative example 1 | 40.1 | 69.8 | 130.1 |

**Test example 2: Activity assay of coagulation factor FXIa inhibitor**

**1. Test sample**

The compounds in examples and comparative example 1.

**2. Test procedure**

1) An assay buffer solution (50 mM HEPES, 5 mM KCl, 145 mM NaCl, 1 mg/ml PEG8000, with a pH of 7.4) was prepared and equilibrated to room temperature;
2) a 10X compound working solution was prepared;
3) a 0.8 nM human FXIa working solution (2X) was prepared and thoroughly mixed for later use;
4) 20 μL of the FXIa working solution in step 3) was added to all the assay wells of a 384-well plate (Coring, 3702) and centrifuged (200 g, RT) for 10 s;
5) 4 μL of the compound working solution in step 2) was added to the corresponding assay wells in the 384-well plate and centrifuged (200 g, RT) for 10 s, and then the working plate was incubated at 25°C for 20 min;
6) a 750 μM S-2366 working solution (2.5X) was prepared and thoroughly mixed for later use;
7) 16 μL of the S-2366 working solution in step 6) was added to all the assay wells in a 384-well plate and centrifuged (200 g, RT) for 10 s, and then the working plate was incubated at 37°C for 45 min; and
8) after incubation, the absorbance OD405nm was read using EnVision, and the data were collected.

**3. Data analysis**

1)

$$Z' \text{ factor} = 1 - 3*(SD_{Max} + SD_{Min})/(Mean_{Max} - Mean_{Min});$$

2)

$$CV_{Max} = (SD_{Max}/Mean_{Max})*100\%;$$

3)

$$CVMin = (SD_{Min}/Mean_{Min})*100\%;$$

4)

$$S/B = Singal/Background;$$

5) blank control: 0.1% DMSO; positive control: comparative example 1;

6)

$$\text{calculation formula for } IC_{50}\text{: } Y = Bottom + (Top - Bottom)/(1 + 10\^{}((LogIC_{50} - X)*HillSlope))$$

X: log value of compound concentration; Y: inhibition rate (%).

Concentration setting: 5 concentrations: 200 nM, 40 nM, 8 nM, 1.6 nM and 0.32 nM, and $IC_{50}$ value was detected.

## 4. Test results

The test results are shown in the table below: All of the tested compounds of the present disclosure have a relatively significant in-vitro inhibitory effect on FXIa, among which compounds 2, 6, 17, 21, 25, 26 and 27 of the present disclosure have a stronger inhibitory effect on FXIa than the compound in comparative example 1, especially for compounds 2 and 6 which exhibit a more significant effect. Other tested compounds of the present disclosure have an inhibitory effect on FXIa comparable to that of the comparative example.

**Table 2 Activity assay of coagulation factor FXIa inhibitor**

| **Sample** | **$IC_{50}$ (nM)** | **Sample** | **$IC_{50}$ (nM)** |
|---|---|---|---|
| Compound 1 | 7.53 | Compound 2 | 0.91 |
| Compound 5 | 5.60 | Compound 6 | 0.96 |
| Compound 9 | 6.32 | Compound 13 | 25.5 |
| Compound 17 | 1.65 | Compound 18 | 9.06 |
| Compound 21 | 2.09 | Compound 22 | 4.23 |
| Compound 25 | 1.87 | Compound 26 | 1.15 |
| Compound 27 | 2.98 | Comparative example 1 | 7.80 |

## Test example 3: Study of pharmacokinetics in rats

## 1. Test sample

Compound in comparative example 1 and compounds 2, 6, 21, 25, 26, 27 and 28 in examples.

## 2. Test operation

In the experiment, 48 male SD rats were divided into 8 groups (6 in each group): a group for comparative example 1, a group for compound 2, a group for compound 6, a group for compound 21, a group for compound 25, a group for compound 26, a group for compound 27, and a group for compound 28, respectively. The rats were fasted for 12 h before administration, with free access to water only. Each group was administered via intragastrical gavage at a dose of 30 mg/kg.

Blank blood samples were collected before administration, and blood samples were collected at the following predetermined time points after administration: 5 min, 15 min, 30 min, 45 min, 1 h, 2 h, 3 h, 5 h, 7 h and 24h. About 0.5 mL of blood was collected, placed in an EDTA-$K_2$ tube, centrifuged to separate plasma, and stored at -80°C. The drug plasma concentration was measured using a liquid chromatography-tandem mass spectrometry analysis system (LC-MS/MS), and pharmacokinetic parameters, such as AUC0-t, Cmax, Tmax and t1/2, were calculated based on the drug plasma concentration data using DAS 2.0 software.

### 3. Test results

The experimental results of the pharmacokinetic study of oral administration in rats are shown in the table below. Compared with comparative example 1, compounds 2, 6, 21, 25, 26 and 27 in the examples have better absorption characteristics, showing significantly improved **$AUC_{(0-t)}$**. Notably, compound 21, compound 25, compound 26, and compound 27 have significantly improved AUC and Cmax relative to comparative example 1 at the same dose, indicating that the compounds in these examples can achieve the same efficacy at a lower dose.

Additionally, compared with comparative example 1 and compound 28, compound 21, compound 25, compound 26, and compound 27 have a shorter half-life and a pharmacokinetic characteristic of rapid elimination, indicating that the compounds have a lower safety risk of potential bleeding. It also demonstrates that not all the deuteration sites have an effect of extending half-life. Notably, the present disclosure unexpectedly found that only deuterated compounds at $R^9$ and/or $R^2$ sites in the structure of general formula (1) have more advantageous pharmacokinetic results.

**Table 3 Pharmacokinetic results**

| Compound | $AUC_{(0-t)}$ (ug/L*h) | $t_{1/2}$ (h) | $T_{max}$ (h) | $C_{max}$ (ug/L) |
|---|---|---|---|---|
| Comparative example 1 | 73,998 | 6.14 | 2.83 | 6,300 |
| Compound 2 | 85,885 | 7.75 | 3.94 | 6,737 |
| Compound 6 | 89,785 | 7.08 | 4.10 | 6,437 |
| Compound 21 | 273,612 | 2.96 | 5.00 | 26,867 |
| Compound 25 | 159,025 | 1.9 | 2.30 | 26,467 |
| Compound 26 | 128,935 | 3.33 | 2.70 | 21,034 |
| Compound 27 | 290,874 | 4.01 | 4.43 | 27,189 |
| Compound 28 | 70,918 | 5.88 | 5.13 | 6,222 |

## Test example 4: Effect of continuous in-vivo administration on general condition and food intake of rats

### 1. Test drug

Comparative example 1, and compound 2, compound 21, compound 25, compound 26 and compound 27 in examples.

### 2. Test scheme

70 healthy adult SD rats were randomly divided into 7 groups: a blank group, a group for comparative example 1, a group for compound 2, a group for compound 21, a group for compound 25, a group for compound 26, and a group for compound 27. Each group had 10 rats, equally distributed by gender. The rats received drug administrations via oral gavage once a day for 14 consecutive days, following the administration regimen outlined in the table below. The blank group was given an equal volume of a vehicle. After administration, the effects of the drugs on the general condition and food intake of rats were observed and recorded.

**Table 4 Continuous administration regimen**

| Experiment grouping | Test substance | Number of animals | Administration dose (mg/kg) | Dosing volume (ml/kg) | Route of administration |
|---|---|---|---|---|---|
| Blank group | Vehicle | 10 | / | 10 | PO |
| Group for comparative | Comparative example 1 | 10 | 500 | 10 | PO |
| example 1 | | | | | |
| Group for compound 2 | Compound 2 | 10 | 500 | 10 | PO |
| Group for compound 21 | Compound 21 | 10 | 500 | 10 | PO |

(continued)

| Experiment grouping | Test substance | Number of animals | Administration dose (mg/kg) | Dosing volume (ml/kg) | Route of administration |
|---|---|---|---|---|---|
| Group for compound 25 | Compound 25 | 10 | 500 | 10 | PO |
| Group for compound 26 | Compound 26 | 10 | 500 | 10 | PO |
| Group for compound 27 | Compound 27 | 10 | 500 | 10 | PO |

**3. Test results**

In the study on the general condition of the rats in each group after 14 consecutive days of oral gavage administration, piloerection and hypoactivity were found in 1/3 or more of the rats in the comparative example 1 group; and no apparent abnormalities were observed in the remaining groups.

After 14 days of continuous oral gavage administration, the food intake of the rats in each group was observed. The results are shown in the table below.

**Table 5 Observation results of food intake of rats (within 24 hours after the last administration)**

| **Group** | **Blank group** | **Group for comparative example 1** | **Group for compound 2** | **Group for compound 21** |
|---|---|---|---|---|
| Food intake (g, mean ±SD) | 27.68±1.45 | 24.98±0.92** | 27.22±1.33 | 27.08±1.23 |
| **Group** | **Compound 25** | **Compound 26** | **Compound 27** | |
| Food intake (g, mean ±SD) | 27.00±1.42 | 26.49±1.21 | 27.08±1.19 | |
| Note: **P < 0.01 vs blank group | | | | |

As can be seen from the table above, compared with the blank group, the rats in the group for comparative example 1 showed a significantly reduced food intake (P < 0.001) within 24 hours after the last administration. By contrast, the rats in the groups for compound 2, compound 21, compound 25, compound 26 and compound 27 showed essentially the same food intake as the blank group within 24 hours after the last administration, with no statistically significant difference. The compounds of the present disclosure demonstrate higher safety profiles with reduced tissue toxicity risk.

The examples described above are only one of the preferred embodiments of the present disclosure and should not be construed as limiting the scope of protection of the present disclosure. Any modification or refinement made without substantive departure from the core design concept and spirit of the present disclosure, which still solves technical problems consistent with the present disclosure, shall be included in the scope of protection of the present disclosure.

## Claims

**1.** A compound represented by Formula (I), or a stereoisomer or pharmaceutically acceptable salt thereof:

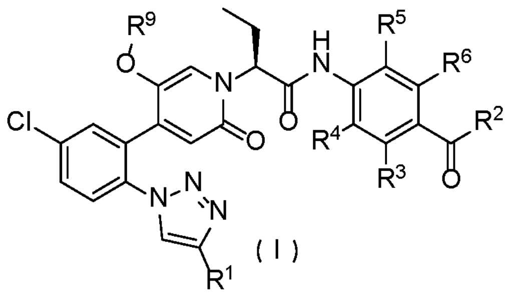

(I)

wherein

$R^1$ is selected from halogen and trifluoromethyl;
$R^2$ is independently selected from $NH_2$, $NHR^7$, OH and $OR^8$, wherein $R^7$ and $R^8$ are selected from alkyl and cycloalkyl, and the hydrogen of $R^7$ and $R^8$ may be substituted with one or more deuterium;
$R^3$ and $R^6$ are independently selected from hydrogen, halogen, alkoxy, haloalkyl and $CONH_2$;
$R^4$ and $R^5$ are independently selected from hydrogen, halogen, alkoxy and haloalkyl; and
$R^9$ is independently selected from $CH_3$ and $CD_3$;
wherein the following compounds are excluded:

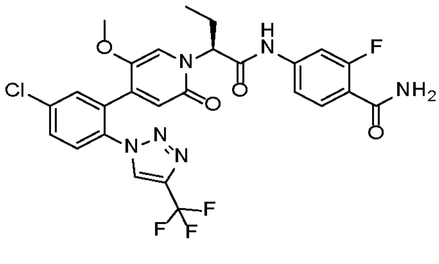,
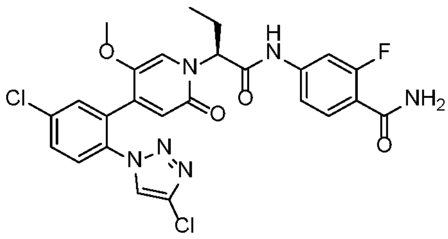,

and

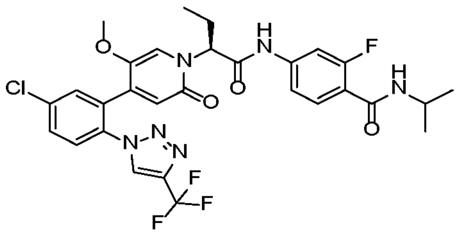.

**2.** The compound of claim 1, or a stereoisomer or pharmaceutically acceptable salt thereof, wherein

$R^1$ is selected from chloro or trifluoromethyl; and/or
$R^2$ is independently selected from $NH_2$, $NHR^7$, OH and $OR^8$, wherein $R^7$ and $R^8$ are selected from methyl, ethyl, propyl, cyclopropyl, cyclopropylmethyl and tert-butyl, and the hydrogen of $R^7$ and $R^8$ may be substituted with one or more deuterium; and/or
$R^3$ and $R^6$ are independently selected from hydrogen, fluoro, chloro, methoxy, trifluoromethyl and $CONH_2$; and/or
$R^4$ and $R^5$ are independently selected from hydrogen, fluoro, chloro, methoxy and trifluoromethyl; and/or
$R^9$ is independently selected from $CH_3$ and $CD_3$.

**3.** The compound of claim 1, or a stereoisomer or pharmaceutically acceptable salt thereof, wherein

$R^1$ is trifluoromethyl; and/or
$R^2$ is independently selected from $NH_2$, $NHR^7$, OH and $OR^8$, wherein $R^7$ and $R^8$ are selected from methyl, ethyl, propyl, cyclopropyl, cyclopropylmethyl and tert-butyl, and the hydrogen of $R^7$ and $R^8$ may be substituted with one or more deuterium; and/or
$R^3$ and $R^6$ are independently selected from hydrogen, fluoro, chloro, methoxy, trifluoromethyl and $CONH_2$; and/or
$R^4$ and $R^5$ are independently selected from hydrogen, fluoro and chloro; and/or
$R^9$ is independently selected from $CH_3$ and $CD_3$.

**4.** The compound of any one of claims 1-3, or a stereoisomer or pharmaceutically acceptable salt thereof, wherein

$R^1$ is trifluoromethyl; and/or
$R^2$ is independently selected from $NH_2$, $NHCH_3$, $NHCD_3$, OH, $OCH_3$, $OCD_3$ and $OC(CH_3)_3$; and/or
$R^3$ and $R^6$ are independently selected from fluoro, chloro, methoxy and trifluoromethyl; and/or
$R^4$ and $R^5$ are independently selected from hydrogen, fluoro and chloro; and/or
$R^9$ is independently selected from $CH_3$ and $CD_3$.

**5.** The compound of any one of claims 1-4, or a stereoisomer or pharmaceutically acceptable salt thereof, wherein the compound is selected from the following structures:

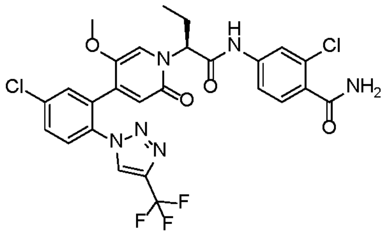
,
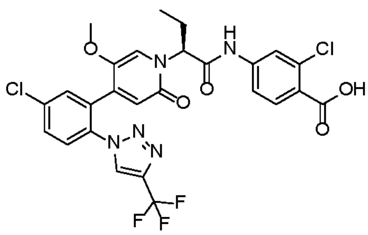
,
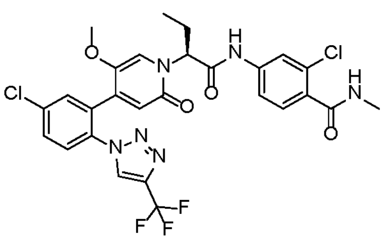
,
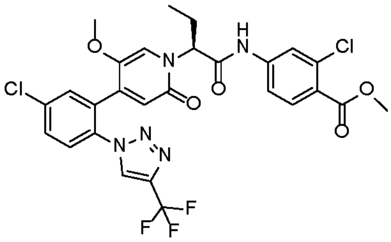
,
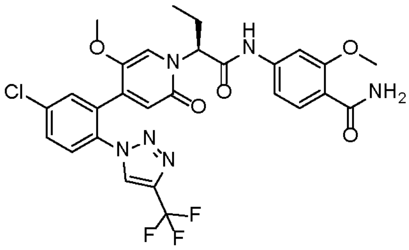
,
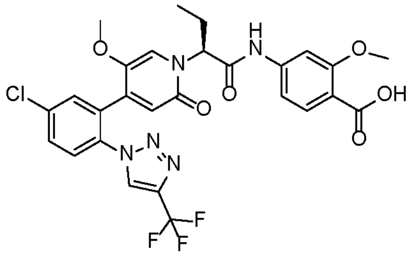
,
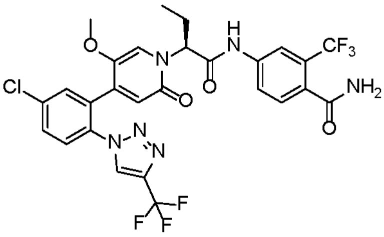
,
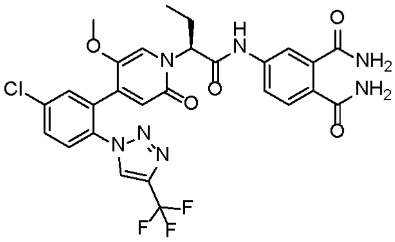
,
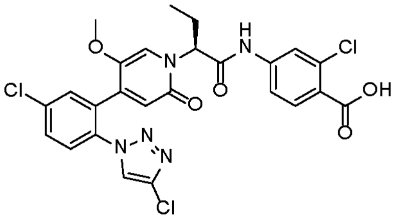
,
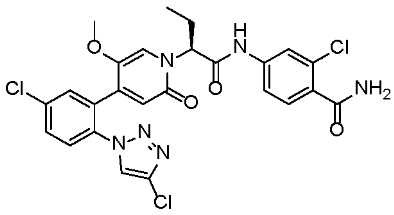
,
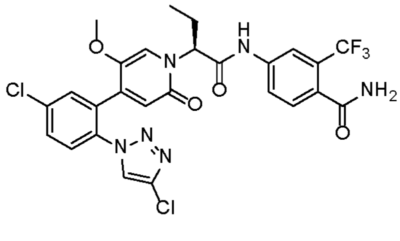
,
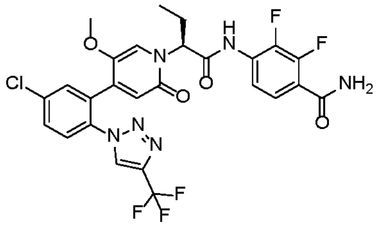
,

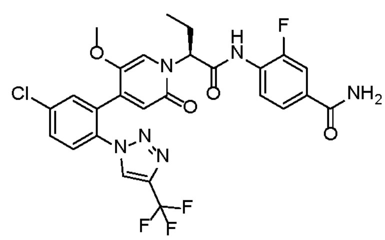,
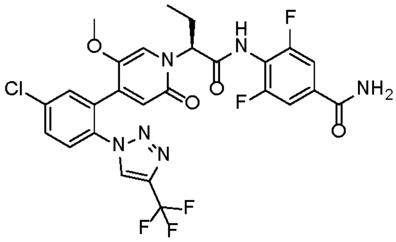,

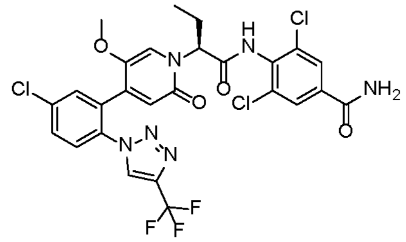,
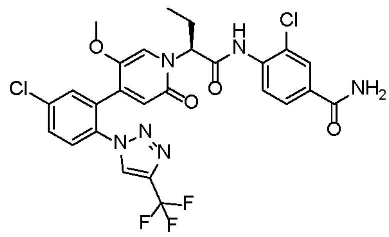,

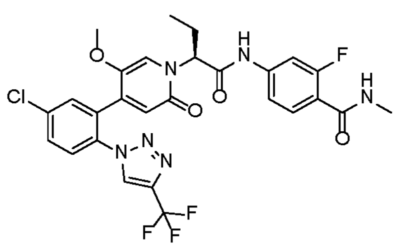,
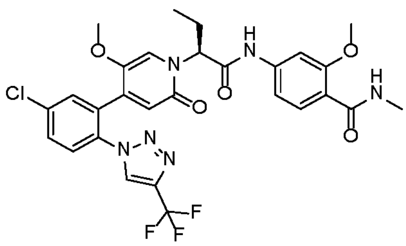,

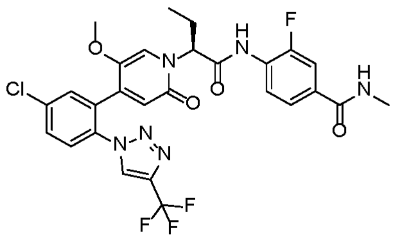,
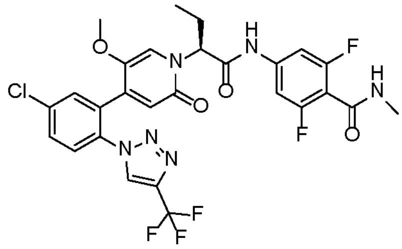,

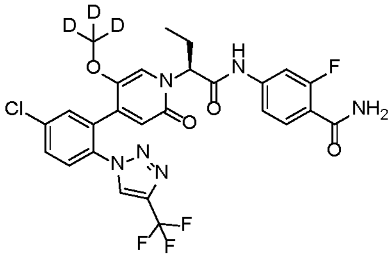,
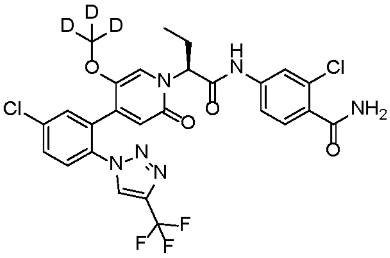,

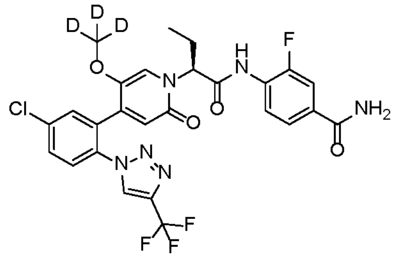,
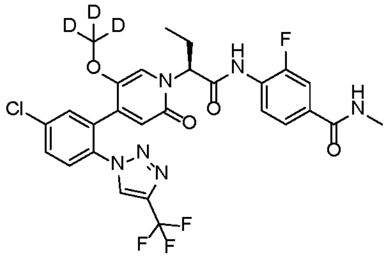,

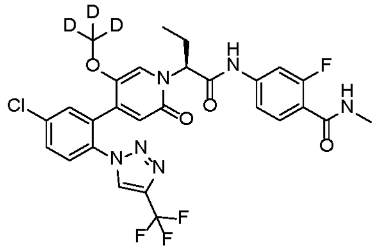,
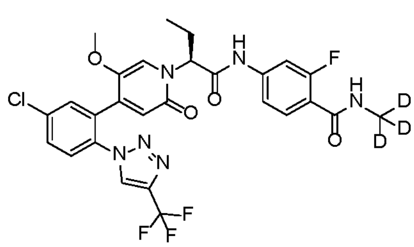,

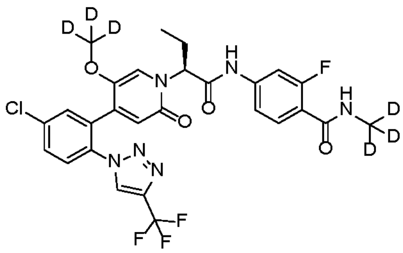,
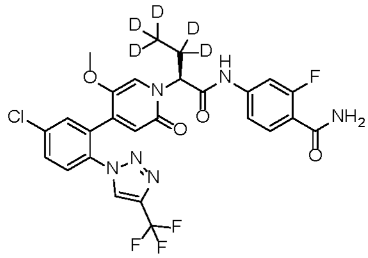,

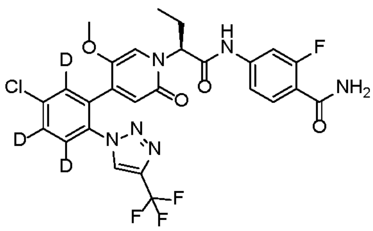
and
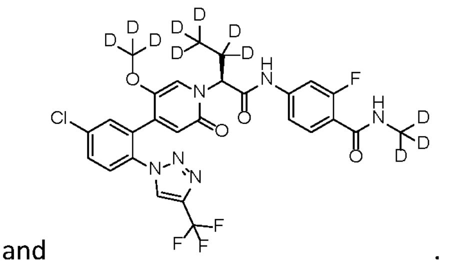.

6. The compound of any one of claims 1-5, or a stereoisomer or pharmaceutically acceptable salt thereof, wherein the hydrogen in the compound or the stereoisomer or pharmaceutically acceptable salt thereof may be substituted with one or more deuterium.

7. The compound of any one of claims 1-6, or a stereoisomer or pharmaceutically acceptable salt thereof, wherein the compound is prepared by a method comprising the following steps:

when $R^2$ is $NH_2$, $NHR^7$ or $OR^8$, the synthesis route is as follows:

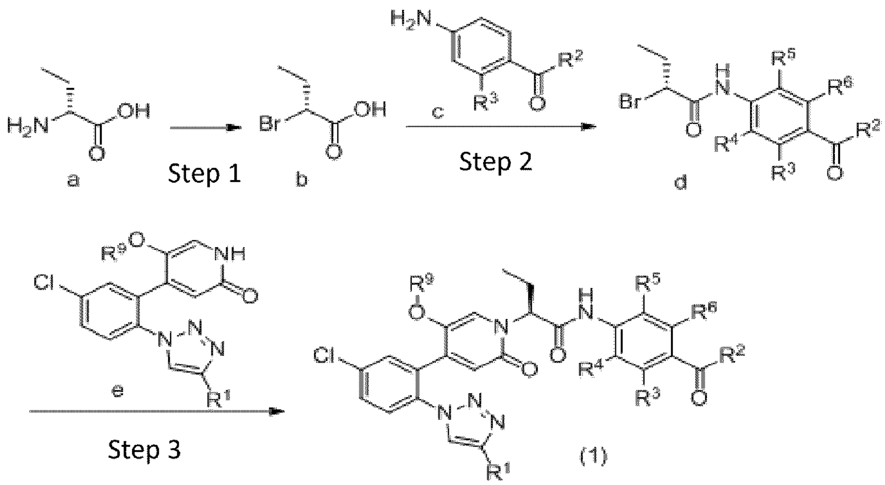


wherein $R^1$, $R^3$, $R^4$, $R^5$, $R^6$, $R^7$, $R^8$ and $R^9$ are defined the same as those in any one of claims 1-6;
step 1: bromination of starting material a to produce intermediate b;
step 2: condensation of intermediate b with starting material c to produce intermediate c; and

step 3: a substitution reaction of intermediate d with compound e to produce a compound of Formula (1); and when $R^2$ is OH, the synthesis route is as follows:

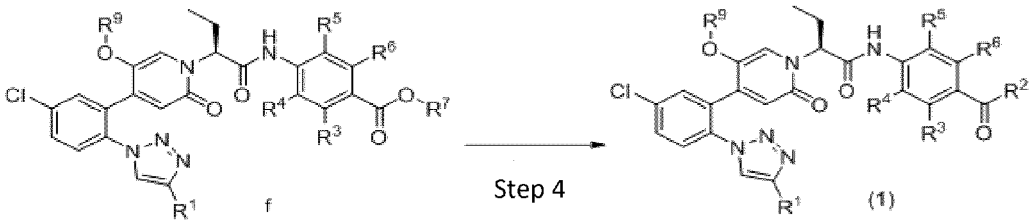


f

(1)

wherein $R^1$, $R^3$, $R^4$, $R^5$, $R^6$, $R^7$ and $R^9$ are defined the same as those in any one of claims 1-6;
step 4: hydrolysis of compound f under a basic condition to produce a compound of Formula (1).

**8.** A key intermediate having a structure represented by Formula e below:

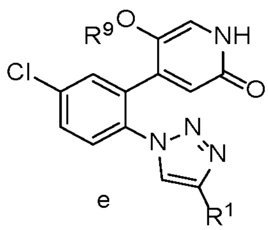

e

,

wherein

$R^1$ is selected from halogen and trifluoromethyl, and $R^9$ is selected from $CH_3$ and $CD_3$;
preferably, $R^1$ is selected from halogen and trifluoromethyl, and $R^9$ is $CD_3$.

**9.** The intermediate of claim 8, wherein the intermediate is selected from the following compounds:

4-(5-chloro-2-(4-(trifluoromethyl)-1H-1,2,3-triazol-1-yl)phenyl)-5-(methoxy-d3)pyridine-2(1H)-one; and
4-(5-chloro-2-(4-chloro-1H-1,2,3-triazol-1-yl)phenyl)-5-(methoxy-d3)pyridine-2(1H)-one.

**10.** A pharmaceutical composition comprising

the compound of any one of claims 1-6, or a stereoisomer or pharmaceutically acceptable salt thereof, and a pharmaceutically acceptable excipient.

**11.** Use of the compound of any one of claims 1-6 or a stereoisomer or pharmaceutically acceptable salt thereof in the preparation of a medicament for treating and/or preventing an FXIa receptor-associated disease.

**12.** The use of claim 9, wherein the FXIa receptor-associated disease is selected from a cerebrovascular arterial disease and/or a peripheral arterial disease; preferably, the FXIa receptor-associated disease is selected from transient ischemic attack (TIA), ischemic stroke, including cardioembolic stroke such as stroke caused by atrial fibrillation, non-cardioembolic stroke such as lacunar stroke, stroke caused by an aortic or arteriolar disease or stroke of undetermined etiology, cryptogenic stroke, embolic stroke, embolic stroke of undetermined source, or an event leading to stroke or TIA and originating from thrombosis and/or thromboembolism, and/or a peripheral arterial condition leading to the peripheral arterial disease, including peripheral arterial occlusion, acute limb ischemia, amputation, reocclusion and restenosis after intervention (for example, angioplasty, stent implantation or surgery, and bypass grafting), and/or stent thrombosis.

## INTERNATIONAL SEARCH REPORT

International application No. **PCT/CN2023/140251**

**A. CLASSIFICATION OF SUBJECT MATTER**

C07D 401/10(2006.01)i; A61K 31/4439(2006.01)i; A61P 9/10(2006.01)i; A61P 9/00(2006.01)i; A61P 7/02(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, WPABS, VEN, CNKI, REGISTRY(STN), CAPLUS(STN): 成都施贝康生物医药科技有限公司, 氧代吡啶, FXIa, pyridin+, one, pyrimidone, pyridone, blood coagulation factor, structural formula search.

**C. DOCUMENTS CONSIDERED TO BE RELEVANT**

| Category* | Citation of document, with indication, where appropriate, of the relevant passages | Relevant to claim No. |
|---|---|---|
| X | CN 108026072 A (BAYER PHARMA AG) 11 May 2018 (2018-05-11) claims 1-13, and embodiments 1-259 | 1-6, 10-12 |
| X | WO 2015120777 A1 (SICHUAN HAISCO PHARMACEUTICAL CO., LTD.) 20 August 2015 (2015-08-20) claims 1-12 | 1-6, 10-12 |
| X | WO 2014160592 A2 (MERCK SHARP & DOHME CORP. et al.) 02 October 2014 (2014-10-02) claims 1-14 | 1-6, 10-12 |
| X | CN 111770917 A (BAYER AG et al.) 13 October 2020 (2020-10-13) description, pages 5-6, paragraph [0015] | 7-9 |
| PX | CN 116082303 A (CHENGDU SHIBEIKANG BIOLOGICAL MEDICINE TECHNOLOGY CO., LTD.) 09 May 2023 (2023-05-09) claims 1-12 | 1-12 |
| PX | CN 116535392 A (CHENGDU SHIBEIKANG BIOLOGICAL MEDICINE TECHNOLOGY CO., LTD.) 04 August 2023 (2023-08-04) abstract, and embodiments 3-7 | 1-6, 8-12 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 March 2024** | **15 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/140251**

**C. DOCUMENTS CONSIDERED TO BE RELEVANT**

| Category* | Citation of document, with indication, where appropriate, of the relevant passages | Relevant to claim No. |
|---|---|---|
| PX | CN 116621728 A (CHENGDU SHIBEIKANG BIOLOGICAL MEDICINE TECHNOLOGY CO., LTD.) 22 August 2023 (2023-08-22) abstract, and embodiments 3-7 | 1-6, 8-12 |
| PX | CN 116621742 A (CHENGDU SHIBEIKANG BIOLOGICAL MEDICINE TECHNOLOGY CO., LTD.) 22 August 2023 (2023-08-22) abstract, and embodiments 3-7 | 1-6, 8-12 |
| PX | CN 116730861 A (CHENGDU SHIBEIKANG BIOLOGICAL MEDICINE TECHNOLOGY CO., LTD.) 12 September 2023 (2023-09-12) abstract, and embodiments 1-8 | 1-6, 8-12 |
| PX | CN 116751136 A (CHENGDU SHIBEIKANG BIOLOGICAL MEDICINE TECHNOLOGY CO., LTD.) 15 September 2023 (2023-09-15) abstract, and embodiments 1-8 | 1-6, 8-12 |
| PX | CN 116874387 A (CHENGDU SHIBEIKANG BIOLOGICAL MEDICINE TECHNOLOGY CO., LTD.) 13 October 2023 (2023-10-13) abstract, and embodiments 1-8 | 1-6, 8-12 |
| PX | CN 116262735 A (CHENGDU SHIBEIKANG BIOLOGICAL MEDICINE TECHNOLOGY CO., LTD.) 16 June 2023 (2023-06-16) embodiment 1, intermediate compounds e and f | 1-4, 6 |
| PX | CN 116903585 A (TIANJIN INSTITUTE OF PHARMACEUTICAL RESEARCH CO., LTD.) 20 October 2023 (2023-10-20) description, paragraph [0129], and intermediates 7 and 3 | 1, 8 |

Form PCT/ISA/210 (second sheet) (July 2022)

**INTERNATIONAL SEARCH REPORT**
**Information on patent family members**

International application No.
**PCT/CN2023/140251**

| Patent document cited in search report | Publication date (day/month/year) | Patent family member(s) | Publication date (day/month/year) |
|---|---|---|---|
| CN 108026072 A | 11 May 2018 | BR 112018000209 A2 | 04 September 2018 |
| | | MA 42376 A | 16 May 2018 |
| | | MA 42376 B1 | 30 April 2021 |
| | | MX 2018000076 A | 26 February 2018 |
| | | JP 2018519323 A | 19 July 2018 |
| | | JP 6871180 B2 | 12 May 2021 |
| | | AU 2016289746 A1 | 25 January 2018 |
| | | AU 2016289746 B2 | 20 August 2020 |
| | | CY 1123996 T1 | 22 July 2022 |
| | | UA 122341 C2 | 26 October 2020 |
| | | MY 196640 A | 25 April 2023 |
| | | JO 3703 B1 | 31 January 2021 |
| | | PH 12018500057 A1 | 09 July 2018 |
| | | NI 201800001 A | 19 October 2018 |
| | | SI 3319956 T1 | 26 February 2021 |
| | | CR 20180017 A | 07 March 2018 |
| | | ZA 201800826 B | 25 August 2021 |
| | | PL 3319956 T3 | 05 July 2021 |
| | | IL 256556 A | 28 February 2018 |
| | | IL 256556 B | 28 February 2021 |
| | | DK 3319956 T3 | 29 March 2021 |
| | | HRP 20210459 T1 | 14 May 2021 |
| | | US 2023024752 A1 | 26 January 2023 |
| | | PE 20180538 A1 | 20 March 2018 |
| | | SV 2018005610 A | 27 April 2018 |
| | | HK 1255045 A1 | 02 August 2019 |
| | | EP 3319956 A1 | 16 May 2018 |
| | | EP 3319956 B1 | 06 January 2021 |
| | | ES 2856554 T3 | 27 September 2021 |
| | | UY 36780 A | 31 January 2017 |
| | | TN 2018000011 A1 | 08 July 2019 |
| | | EA 201890111 A1 | 29 June 2018 |
| | | EA 036208 B1 | 14 October 2020 |
| | | US 2019367478 A1 | 05 December 2019 |
| | | US 11180471 B2 | 23 November 2021 |
| | | KR 20180026761 A | 13 March 2018 |
| | | KR 102596164 B1 | 01 November 2023 |
| | | WO 2017005725 A1 | 12 January 2017 |
| | | HUE 053552 T2 | 28 July 2021 |
| | | CL 2018000039 A1 | 13 July 2018 |
| | | DOP 2018000004 A | 31 January 2018 |
| | | CU 20180001 A7 | 05 June 2018 |
| | | CU 24512 B1 | 12 May 2021 |
| | | US 2018194745 A1 | 12 July 2018 |
| | | US 10421742 B2 | 24 September 2019 |
| | | TW 201706261 A | 16 February 2017 |
| | | TWI 717367 B | 01 February 2021 |
| | | LT 3319956 T | 10 February 2021 |
| | | GEP 20197046 B | 10 December 2019 |
| | | ECSP 18001308 A | 31 March 2018 |

Form PCT/ISA/210 (patent family annex) (July 2022)

**INTERNATIONAL SEARCH REPORT**
**Information on patent family members**

International application No.
**PCT/CN2023/140251**

| Patent document cited in search report | Publication date (day/month/year) | Patent family member(s) | Publication date (day/month/year) |
|---|---|---|---|
| | | RS 61584 B1 | 29 April 2021 |
| | | CA 2990901 A1 | 12 January 2017 |
| | | CO 2018000113 A2 | 12 June 2018 |
| WO 2015120777 A1 | 20 August 2015 | US 2017029423 A1 | 02 February 2017 |
| | | US 10174020 B2 | 08 January 2019 |
| | | EP 3138839 A1 | 08 March 2017 |
| | | EP 3138839 A4 | 03 January 2018 |
| | | EP 3138839 B1 | 28 October 2020 |
| WO 2014160592 A2 | 02 October 2014 | US 2016046581 A1 | 18 February 2016 |
| | | US 9809545 B2 | 07 November 2017 |
| | | WO 2014160592 A3 | 24 December 2014 |
| | | EP 2978425 A2 | 03 February 2016 |
| | | EP 2978425 A4 | 05 October 2016 |
| | | EP 2978425 B1 | 27 September 2017 |
| CN 111770917 A | 13 October 2020 | ES 2920449 T3 | 04 August 2022 |
| | | RU 2020133706 A | 18 April 2022 |
| | | IL 277240 A | 29 October 2020 |
| | | IL 277240 B1 | 01 September 2023 |
| | | IL 277240 B2 | 01 January 2024 |
| | | US 2021017152 A1 | 21 January 2021 |
| | | US 11542245 B2 | 03 January 2023 |
| | | UA 126940 C2 | 22 February 2023 |
| | | EP 3765452 A1 | 20 January 2021 |
| | | EP 3765452 B1 | 13 April 2022 |
| | | TW 202002969 A | 16 January 2020 |
| | | EP 3889147 A1 | 06 October 2021 |
| | | JP 2021517573 A | 26 July 2021 |
| | | JP 7287978 B2 | 06 June 2023 |
| | | LT 3765452 T | 25 May 2022 |
| | | AU 2019236369 A1 | 30 July 2020 |
| | | PE 20211395 A1 | 27 July 2021 |
| | | MX 2020009580 A | 05 October 2020 |
| | | CA 3093733 A1 | 19 September 2019 |
| | | SG 11202006710 RA | 28 August 2020 |
| | | BR 112020018562 A2 | 29 December 2020 |
| | | UY 38147 A | 01 October 2019 |
| | | WO 2019175043 A1 | 19 September 2019 |
| | | KR 20200131241 A | 23 November 2020 |
| | | IL 300463 A | 01 April 2023 |
| | | CL 2020002371 A1 | 29 January 2021 |
| | | PT 3765452 T | 23 June 2022 |
| | | JOP 20200232 A1 | 14 September 2020 |
| | | JOP 20200232 B1 | 17 September 2023 |
| | | HRP 20220565 T1 | 10 June 2022 |
| | | AR 117400 A1 | 04 August 2021 |
| | | RS 63338 B1 | 29 July 2022 |
| | | SI 3765452 T1 | 30 June 2022 |
| | | HUE 058796 T2 | 28 September 2022 |
| | | PL 3765452 T3 | 01 August 2022 |
| | | DK 3765452 T3 | 27 June 2022 |

Form PCT/ISA/210 (patent family annex) (July 2022)

**INTERNATIONAL SEARCH REPORT**
**Information on patent family members**

International application No.
**PCT/CN2023/140251**

| Patent document cited in search report | Publication date (day/month/year) | Patent family member(s) | Publication date (day/month/year) |
|---|---|---|---|
| CN 116082303 A | 09 May 2023 | None | |
| CN 116535392 A | 04 August 2023 | None | |
| CN 116621728 A | 22 August 2023 | None | |
| CN 116621742 A | 22 August 2023 | None | |
| CN 116730861 A | 12 September 2023 | None | |
| CN 116751136 A | 15 September 2023 | None | |
| CN 116874387 A | 13 October 2023 | None | |
| CN 116262735 A | 16 June 2023 | None | |
| CN 116903585 A | 20 October 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211647390
- CN 108026072 B


### Non-patent literature cited in the description


- **SCOTT L. HARBESON** ; **ROGER D. TUNG.** *Deuterium in Drug Discovery and Development*, 405-406